# EUROPEAN PATENT SPECIFICATION

(11) **EP 0 495 187 B1**
(45) Date of publication and mention of the grant of the patent: **22.01.1997**
(21) Application number: 91120485.7
(22) Date of filing: 29.11.1991
(51) Int. Cl.: A61K 9/51, A61K 9/16

(54) **Protein nanomatrixes and method of production**
Protein Nanomatrizen und Verfahren zur Herstellung
Nanonmatrices protéiniques et méthode de production

(30) Priority: 15.01.1991 US 641270
(43) Date of publication of application: 22.07.1992
(73) Proprietor: HEMOSPHERE, INC., Irvine California 92715 (US)
(72) Inventor: Yen, Richard C.K., Glendora California 91740 (US)
(74) Representative: Baillie, Iain Cameron

(56) References cited:
- DE-A- 1 916 704
- GB-A- 1 516 348
- US-A- 4 671 954
- JOURNAL OF PHARMACEUTICAL SCIENCES vol. 71, no. 7, July 1982, pages 759 - 762; LEVY M.-C. ET AL: 'Microencapsulation IV:Cross-linked Hemoglobin Microcapsules'

## Description

The present invention relates to the field of composition for delivery of information and bioreactive molecules to cultured cells or live animals by encapsulation within and/or adhesion on the surface of microscopic nanomatrixes and methods of synthesis.

Information molecules are molecules, such as most DNA and RNA polynucleotides, which store information for the synthesis of other biological molecules which have direct effects on the structure and/or function of biological systems. Bioreactive molecules are molecules (e.g. antibiotics, enzymes, cytokines, receptors, hormones, immunoglobulins and hemoglobins) which can directly affect biological systems and they can either be synthesized by natural biological systems or synthesized artificially (e.g. synthetic drugs and immunoglobulin-toxin conjugates).

The distinction between these two basic types of molecules, however, is not always absolute and distinct. For example, certain RNA species, such as ribozymes, are themselves RNA molecules by structure but possess enzymatic activities which allow them to cut other RNA molecules such as those carrying the Human Immunodeficiency Virus (HIV) genetic information. In this patent application the distinction is made for these two basic classes of molecules arbitrarily because the requirement of nanomatrix structure most suitable for delivering one kind of molecules may not be optimal for the other. Minor modifications of the basic synthesis method is disclosed here, which may facilitate the delivery of one kind of molecules more than the other.

The prior art references listed below will be cited in the following discussion:
1. United States Patent No. 4,269,821 issued to Kreuter et al. on May 26, 1981 for "Biological Material" (hereafter the "Kreuter Patent").
2. United States Patent No. 4,107,288 issued to Oppenheim et al. on August 15, 1978 for "Injectable Compositions, Nanoparticles Useful Therein, And Process of Manufacturing Same" (hereafter the "Oppenheim Patent").
3. United States Patent No. 3,663,685 issued to Evans et al. on May 16, 1972 for "Biodegradable Radioactive Particles" (hereafter the "Evans Patent").
4. Hoffman, (1991) "New Vector Delivers Genes to Lung Cells", 252 Science 374 (1991) (hereafter the "Hoffman Article").
5. Rose, A New Cationic Linosome Reagent Mediating Nearly Quantitative Transection of Animal Cells, 10 BioTechniques 520 (1991) (hereafter the "Rose Article").
6. McQuade, A Synthetic HIV-1 Protease Inhibitor with Antiviral Activity Arrest HIV-like Particle Maturation, 247 Science 454 (1990) (hereafter the "McQuade Article").
7. Goldsmith, Midwest Symposium Seeks Therapeutic Answers to Global AIDS Problem, 263 J. American Medical Assoc. 345 (1990) (hereafter the "Goldsmith Article").
8. Gendelman, The Macrophage in the Persistence and Pathogenesis of HIV infection, 3 AIDS 475 (1989) (hereafter the "Gendelman Article").
9. Salahuddin, Human T Lymphotropic Virus Type III Infection of Human Alveolar Macrophages, 68 Blood 281 (1986) (hereafter the "Salahuddin Article").
10. Widder, Magnetically Responsive Microspheres and Other Carriers for the Biophysical Targeting of Antitumor Agents, 16 Advances in Pharmacology and Chemotherapy 213 (1979) (hereafter the "Widder Article").

Genetic defects have long been the source of human suffering to which conventional treatment at best offered only minor amelioration of the symptoms without the possibility of cure. Even with the isolation of genes and construction of these gene copies in the laboratory, delivery of such information molecules to cultured cells and live animals remain problematic. Introduction of genetic material to cultured cells by calcium phosphate precipitation or electric shock (electroporation) methods resulted only in very small amount of uptake of the information molecules. In addition, these methods cannot be used on live animals. Attempts to inject DNA or RNA directly to the live animals will result in prompt degradation of these molecules by the enzymes present in the host's serum.

Moderate success has been claimed by scientists using incapacitated viral RNA as vectors to introduce desirable genetic information to cultured cells and live animals. However, the danger of these incapacitated RNA recombining with residual viruses in the host to become replicative is real and forms a major risk in human application. See the Hoffman Article. Other investigators used liposomes as carriers on which surface the information molecules can stick on and enter the cell when the liposomes are phagocytosed. See the Rose Article. Nevertheless, the number of DNA or RNA copies that can attach to the surface of the liposome are few and the possibility of degradation by enzymes are great before the DNA can enter the desired cells.

For the information molecules to be successfully integrated with the host genetic system, multiple hurdles must be overcome. The carrier must carry multiple copies of the information molecules so that enough is left to do their job after some degradation during the journey from injection site to the desired cell. The carrier must be taken up by the cells without breaking the information molecules. The uptake process may be specific in that antibodies pre-bonded to the surface of the carrier can find specific cell surface markers to attach to and deliver the information molecules preferentially to only the desired cell type. Other homing devices may be used, e.g. the purified HIV envelop glycoprotein, gp120, designed to attach to the CD4 receptor of T4 lymphocytes, or the reverse. See the Goldsmith Article. Alternatively, the size and surface properties of the carrier themselves (without homing devices) may be attractive enough for phagocytosis by monocytes and macrophages and therefore deliver biological molecules preferentially to these phagocytic cells. After entry into the cell, the information molecules must be released from the carrier, allowed to integrate with the host DNA, and subsequently transcribed into RNA (e.g. HIV viral material or messenger RNA) and then the information translated into protein structures.

To satisfy these complex conditions, there exists the need for a carrier which can protect the information molecules by encapsulation within its matrix, and also allow binding of these information molecules on the surface of the matrix. It is not known at present how fast the information molecules must be released after entry into the cell. Conceivably, surface-bound information molecules may be released faster. But they may be more prone to extracellular degradation, or intracellular degradation such as within the endosomes. Information molecules trapped within the interior of the carrier, on the other hand, may be released only after the protein matrix of the carrier has been stripped. It is not known if these information molecules would be released just in time, or released too late, if the sorting mechanism of the endosome system has already destined the carrier to the lysosomal degradative pathway. Therefore, there are distinct advantages to having a carrier system where the information molecules can both be carried internally and on the surface. In addition, the stability of the matrix itself is important. Too rigid a structure will hinder the release of the information molecule intracellularly. Too fragile a structure will result in breakdown of the delivery system before entry into the desired cells. The nanomatrixes in this application are designed to have variable degrees of rigidity and are capable of carrying information molecules both internally and externally.

Delivery of bioreactive molecules, whether conventional drugs, enzymes, or other naturally occurring biological molecules, to specific cell types has great medical and scientific appeal. Prior arts include the synthesis of liposomes and cross-linked microspheres. Liposomes are made of hydrophobic fatty material forming single layer or multiple layers to entrap biological material within. Stability of the liposome is a problem and leakiness of entrapped material is another problem. Attempts to add homing devices to liposomes are not particularly successful. Microspheres have been synthesized mainly by emulsification of dissolved protein solutions (e.g. by sonication) to form small droplets after which they are stabilized by heat or chemical cross-linkage. See the Widder Article. It was difficult to obtain uniform size distribution or monodispersity because individual microspheres can vary in size or stick to each other during the heating step or chemical cross-linkage. Alternatively, microspheres are formed by first creating an unstable concentration of protein molecules in a barely soluble state and followed by polymerization of individual molecules into a mass which then falls out of solution as a cross-linked microsphere. The cross-linking agent is absolutely needed to start and continue the polymerization process without which no microspheres will form, and whose activity must be neutralized to stop the reaction. See the Oppenheim Patent.

It is desirable to synthesize a new carrier which has the ability to co-carry both information molecules and bioreactive molecules. One of the bioreactive molecules which may be contemplated is any enzyme which will facilitate the integration of the delivered information molecule onto the host genetic system, e.g. reverse transcriptases which can turn RNA information molecules into DNA for subsequent integration into the host DNA genetic system. Other bioreactive molecules would be drugs which would damage other targets. For example, after infection of the human lymphocyte, the HIV will require cleavage of certain HIV polypeptides for successful packaging of its RNA genetic material. See the McQuade Article. Co-carriage of a drug against the cleavage step together with the ribozyme against the HIV genome may have synergistic effects and may lead to complete intracellular destruction of the invading virus. There is at present no known method to target drugs or information molecules into the interior of infected cells where the HIV hides, far less the ability of co-delivery of two different kinds of attack strategies. See the Salahuddin Article and the Gendelman Article.

Since only a minority of cells are in the right condition to incorporate information molecules, detection and isolation of the cells that have successfully incorporated them remain a high priority. Co-delivery of two or more information molecules (e.g. DNA for the beta-galactosidase gene and DNA for the ribozyme gene incorporated within a single carrier) would allow detection of even one single cell which had the right condition to take up the carrier: expression of the beta-galactosidase gene will give the cell a blue color when incubated in the right substrate, which allows that cell to be isolated from among thousands of other beta-galactosidase negative cells and analysis can be performed later for the expression of the ribozyme activity. This will alleviate the need to synthesize a combination DNA sequence containing both the beta-galactosidase gene and the ribozyme gene.

Encapsulation of bioreactive molecules in nanomatrixes also has the advantage of modifying the side effect profile of the molecules. It is well known that peak serum levels are achieved within a short time of intravascular injection of any bioreactive molecules e.g. drugs. Toxic effects can result from such high serum levels, especially if the drug is given as a bolus injection. To avoid such high concentrations, drugs can be given slowly as a continuous drip. However, the latter method would require prolonged nursing care and possibly even hospitalization with its associated cost. Administration of bioreactive molecules inside nanomatrixes or on their surfaces would allow bolus intravenous injections but gradual release of the molecules (e.g. interferon, interleukin, tumor necrosis factors, growth factors) inside the intravascular compartment.

The half-life of the nanomatrixes or capsules in the blood stream is an important factor. Ideally, different kinds of nanomatrixes or capsules should be available so that some have a longer in vivo half-life than others. Most capsules are expected to be taken up primarily by the reticula-endothelial system (RES), e.g. in the liver and spleen. Alternatively, if the capsules are reasonably stable and also small enough so that the phagocytic cells (e.g. macrophage) do not preferentially ingest them, the capsules would escape the RES long enough to perform other tasks. One such interesting and medically useful task would be targeting of drugs to specific population of cells. The targeting of specific cell types would be carried out by capsules which had antibodies (or other ligands with affinity to specific cell surface receptors) attached on the surface of the capsules directed against antigenic sites of these cells. Higher concentrations of drugs near the surface of the targeted cells and lower systemic side-effects would be desirable benefits from this approach.

Other examples for homing devices include anti-idiotypic antibodies which will attach to other antibodies (which now become the "antigen"), or receptors e.g. CD₄ to attach to the envelop protein gp120 on the HIV virus. Other biological molecules can also be used, e.g. transferrin, mannosylated ligands, asialoglycoproteins, alpha 2-macroglobulin, asialofetuin and aggregated antibodies.

In contrast, capsules with short in vivo half-life because they are designed to redisperse quickly would allow quick dispersal of the encapsulated drugs. Quick dissolution of the carrier prevents abnormally high concentrations of the drugs in the RES organs, where the macrophage tend to ingest particular matters such as the carrier capsules. The reversibility of the capsules allows distribution of the drugs in a pattern more or less similar to the same drugs given via the conventional soluble form even though the drugs are now given as a bolus in the encapsuled form.

Encapsulation of biological agents or biologically interactive ingredients are useful in other medical applications. For example, tiny air bubbles can create a strong contrast of the blood vessels (and the organs within which the blood vessels traverse) against the background during ultrasonography. However, the tiny air bubbles, if injected via a peripheral vein, must travel through the right heart, the pulmonary vasculature and then the left heart before they can reach to the other internal organs. Tiny air bubbles are inherently unstable and so they will not be able to stay in the required physical condition for effective ultrasonographic contrast by the time the intended organs are reached. Encapsulation of the small air bubbles in small capsules will allow the air bubbles to serve their intended function even after long distances of travel within the intravascular compartment.

Similar advantages can be conferred to contrast material for CAT scans and nuclear magnetic resonance (NMR) scans. By encapsulation of the contrast material during injection, the injection site will not have an abnormally high concentration of the contrast material leading to false interpretation of the results. However, the reticula-endothelial system (RES), such as the liver and the spleen, with the many phagocytic cells there, tend to phagocytize particulate matter. If the goal of the contrast study is not aimed at organs of the RES, the capsules should be easily reversible so that the contrast material can be released and the capsules dissolved before the contrast material is concentrated in the RES organs. On the other hand, uptake of contrast material such as paramagnetic metal ion chelate (e.g. Fe₃O₄ and Gadolinium-DTPA chelate) by the RES would lead to digestion of capsule whereby the contrast agent is released for enhancement of the organs during magnetic resonance imaging.

From the above discussion it is clear that nanomatrixes with various degrees of rigidity or reversibility extracellularly or intracellularly offer many advantages not available with prior art capsules which generally take the form of liposome or microsphere synthesis.

In liposomes, a shell is formed by a lipid layer or multiple lipid layers surrounding a central hydrophilic solution containing the medication. The lipid layers are inherently unstable and much research went into stabilizing them during the manufacturing process to prolong shelf life. Once stable liposomes are formed, they do not readily redissolve within the blood stream. It is difficult to design liposomes with various degrees of rigidity or reversibility. In addition, the lipid layer(s) serve as a barrier to diffusion. It is difficult for hydrophilic substrate to diffuse through the hydrophobic layers into the interior of the liposomes, or conversely, for the drugs to get out without physical destruction of the lipid layer(s).

Microspheres, in contrast to liposomes, do not have a surface membrane or a special outer layer to maintain its intactness. Most microspheres are more or less homogenous in structure. To maintain the stability of the microspheres, the manufacturing process in prior art always includes a cross-linking process (by chemical means or by heat denaturation) to stabilize the microspheric mass.

The Oppenheim Patent discloses a process of making microspheres of cross-linked macromolecules by using cross-linking agents such as an aldehyde hardening agent (e.g. glutaraldehyde) to initiate and complete the polymerization process. In addition to the hardship in controlling the sizes of the microspheres formed, the Oppenheim process also produces many aggregations which are very undesirable for the purpose of an in vivo medication carrier.

The Kreuter Patent discloses processes for preparation of submicroscopic particles of physiologically acceptable polymer associated with a biologically active material by using a cross-linking agent such as a polymerisable material soluble in a liquid medium (e.g. methyl methacrylate).

Typically, such processes as disclosed by the Oppenheim Patent and the Kreuter Patent, require the irradiation or heat or the presence and chemical reaction of a cross-linking agent to polymerize the "monomers" (which are the individual protein molecules such as human serum albumin or gelatin molecules) so that the resultant "polymers" will increase in molecular weight. When the mass has reached to the point that the solution cannot hold it in its soluble form, the mass will precipitate as a solid more-or-less spherical form which is the microsphere. The covalent bonding of the "monomers" into a "polymer" by the cross-linking agent provides the stability of the microsphere, but it also means that the microspheres will not be easily reversible without destruction of these stable covalent bonds.

The Evans Patent discloses a method of preparing biodegradable radioactive particles by using heated water-oil solutions.

The Widder Article discloses emulsion polymerization methods of preparation of albumin microspheres (Id., pp. 233-235) and preparation of magnetically responsive albumin microsphere (Id., pp. 241-250). The methods essentially involve the processes of emulsification and heat denaturation of a water-oil solution to produce and stabilize microspheres. The Widder Article has also mentioned that for heat sensitive drugs the microspheres are stabilized by chemical cross-linking.

Such methods as disclosed in the Widder Article and the Evans Patent, uses heat to cross-link and to stabilize the protein mass. Essentially, the emulsion consisting of microscopic protein droplets are heated in oil so that the proteins are denatured as a microscopic particle and stayed that way upon cooling and removal of the oily compounds. These microspheres are extremely sturdy and do not easily reverse into its natural monomeric states. Even after enzymatic digestion of these microspheres by the host, the protein molecules have been irreversibly denatured and rendered "foreign" to the host body.

The essential process of all prior art methods of producing microsphere synthesis as medication carriers is the heating or adding of a chemical cross-linking agent for the cross-linking process. The major disadvantage of these prior art methods is that the drug capsules are hard to undergo the reverse encapsulation process and the high possibility of introducing undesirable new antigenicity to the starting material. It is highly desirable to have an efficient synthetic method which does not involve the using of cross-linking agents nor heating, and yet permit post-synthesis stabilization to various degrees via addition of different kinds of or variable concentrations of cross-linking agents, to thereby produce an effective encapsulation product which is reversible to various degrees and without new antigenicity.

Although it is generally known that addition of water-miscible neutral organic solvents can decrease the solubility of most proteins in water to such an extent that they will precipitate out of solution (A.L. Lehninger, "Biochemistry", Worth Publishers, Inc., p. 134), the prior art does not teach how to desolubilize the protein molecules in a controlled manner so that useful spherical particles can result (which does not further aggregate) instead of useless insoluble random aggregates incompatible with in vivo applications.

It is also generally known that addition of solvents such as alcohol to aqueous dispersions tends to coagulate them (Remington's Pharmaceutical Sciences, Ed: A. R. Gennaro, 1985). Even though albumin molecules are completely soluble in aqueous media, albumin solutions have been interpreted as colloidal "suspensions" because the individual molecules are particles greater than 10 angstrom size and therefore can be regarded as solids of such a size (colloidal range) suspended in an aqueous medium (a "sol") (Remington: p. 271 col 2, lines 13-16). However, the coagulation by prior art is uncontrolled and inevitably results in useless aggregates. In contrast, the process of the present invention leads to monodispersed nanomatrixes which have well defined sizes and properties, which in turn remain as a stable suspension over a long time without changing size or shape or property, even though there is still protein material left in the solution to allow further protein-protein interactions that can promote further aggregation to useless coagulates.

Remington also teaches that "precipitation or gelation (of most hydrophilic sols) can be reversed, and the polymer redissolved by removing the salt through dialysis or by adding more water (p.292, col 1, lines 33-35)." In contrast, the product of the present invention clearly is not reversed by removal of salt through dialysis or by adding more water.

The present invention provides a method of producing monodispersed nanomatrixes for biologically active molecules to bind to or be trapped therein, useful for carrying medication for in vivo administration, which nanomatrixes are stable in the synthetic media but are readily reversible to soluble protein molecules after in vivo administration, the method comprising:
a. dissolving first protein molecules in a buffer of suitable osmolarity between approximately 1 to 970±100 milliosmos, where the first protein molecules are globin protein molecules;
b. adding second protein molecules into said buffer containing said first protein molecules, such that the weight ratio of said first protein molecules and the second protein molecules is approximately 96:4, where the second protein molecules are different from said first protein molecules and selected from the group consisting of albumin protein and denatured hypoallergic protein; and
c. adding a solution containing an organic solvent into said buffer containing said first and second protein molecules, such that the organic solvent is approximately 0.2±0.1 to 2.0±0.1 volume by one volume of said buffer containing said first and second protein molecules, where said organic solvent is an alcohol selected from the group consisting of methanol, ethanol, propanol and butanol;
d. whereby mixing said buffer containing said first and second protein molecules and said solution containing said organic solvent results in a turbid suspension containing monodispersed stable nanomatrixes.

In the present invention method: (a) the first protein ingredient is selected from the globin family and may for example be selected from hemoglobin, internally cross-linked hemoglobin, hemoglobin subunits, polymerized hemoglobin, pyridoxylated-hemoglobin, myoglobin, methemoglobin and recombinant hemoglobins; (b) the second protein ingredient is selected from the albumin family and may for example be selected from human serum albumin, modified albumin, animal serum albumin, and recombinant albumin. A surfactant may also be added; for example the surfactant may be a detergent selected from the group consisting of Anionic, Cationic, Zwitterionic and Non-ionic detergents, where the anionic detergents are Sodium Lauryl Sulfate and Sodium Tetradecyl Sulfate; the surfactant may also be selected from the group of the acid or alkali-treated proteins consisting of gelatin. Preferably the osmolarity is between 1 to 970 plus or minus 100 milliosmos.

The present invention also provides a composition of monodispersed nanomatrixes useful for carrying medication for in vivo administration, where the nanomatrixes are stable in the media in which said nanomatrixes were synthesized but are readily reversible to soluble protein molecules after in vivo administration, the device having a composition of,
a. a first protein ingredient being a globin protein;
b. a second protein ingredient which is different from said first protein ingredient, where the second protein ingredient is albumin protein or denatured hypoallergic protein; and
c. the weight ratio of said first protein ingredient and said second protein ingredient being approximately 96:4.

In one preferred embodiment the invention provides nanomatrices for encapsulating at least one biologically interactive ingredient having a composition of hemoglobin (Hb), human serum albumin (HSA) and at least one biologically interactive ingredient covalently pre-bonded to the protein; where the ratio of the composition of Hb, HSA and the at least one biologically interactive ingredient is essentially 96:4:25.

In another preferred embodiment the invention provides nanomatrices for encapsulating at least one biologically interactive ingredient having a composition of hemoglobin (Hb), human serum albumin (HSA) and at least one biologically interactive ingredient trapped within the nanomatrixes or absorbed on the surface of protein; where the ratio of the composition of Hb, HSA and the at least one biologically interactive ingredient is essentially 96:4:25.

In another preferred embodiment the invention provides nanomatrices for encapsulating at least one biologically interactive ingredient having a composition of essentially 1.5±1% hemoglobin (Hb) and 98.5±1% human serum albumin (HSA).

In another preferred embodiment the invention provides nanomatrices for encapsulating at least one biologically interactive ingredient having a composition of hemoglobin (Hb), human serum albumin (HSA) and at least one biologically interactive ingredient covalently pre-bonded to the protein; where the ratio of the composition of Hb, HSA and the at least one biologically interactive ingredient is essentially 1.5:98.5:25.

In another preferred embodiment the invention provides nanomatrices for encapsulating at least one biologically interactive ingredient having a composition of hemoglobin (Hb), human serum albumin (HSA) and at least one biologically interactive ingredient trapped within the nanomatrixes or absorbed on the surface of protein; where the ratio of the composition of Hb, HSA and the at least one biologically interactive ingredient is essentially 1.5:98.5:25.

In another preferred embodiment the invention provides nanomatrices for encapsulating at least one biologically interactive ingredient having a composition of essentially 96±2% hemoglobin (Hb) and 4±2% gelatin.

In another preferred embodiment the invention provides nanomatrices for encapsulating at least one biologically interactive ingredient having a composition of hemoglobin (Hb), gelatin and at least one biologically interactive ingredient covalently pre-bonded to the protein; where the ratio of the composition of Hb, gelatin and the at least one biologically interactive ingredient is essentially 96:4:25.

In another preferred embodiment the invention provides nanomatrices for encapsulating at least one biologically interactive ingredient having a composition of hemoglobin (Hb), gelatin and at least one biologically interactive ingredient trapped within the nanomatrixes or absorbed on the surface of protein; where the ratio of the composition of Hb, gelatin and the at least one biologically interactive ingredient is essentially 96:4:25.

In another preferred embodiment the invention provides nanomatrices for encapsulating at least one biologically interactive ingredient having a composition of essentially 1.5±1% hemoglobin (Hb) and 98.5±1% gelatin.

In another preferred embodiment the invention provides nanomatrices for encapsulating at least one biologically interactive ingredient having a composition of hemoglobin (Hb), gelatin and at least one biologically interactive ingredient covalently pre-bonded to the protein; where the ratio of the composition of Hb, gelatin and the at least one biologically interactive ingredient is essentially 1.5:98.5:25.

In another preferred embodiment the invention provides nanomatrices for encapsulating at least one biologically interactive ingredient having a composition of hemoglobin (Hb), gelatin and at least one biologically interactive ingredient trapped within the nanomatrixes or absorbed on the surface of protein; where the ratio of the composition of Hb, gelatin and the at least one biologically interactive ingredient is essentially 1.5:98.5:25.

In the above embodiments, the ratio of compositions is the ratio of weights of the compositions.

The method of the present invention may optionally further comprise the following steps either performed individually or in any combination thereof: (a) adding another solution containing a surfactant after adding the second protein ingredient; (b) using at least one biologically interactive ingredient to pre-bond the protein ingredient; and/or (c) separating the nanomatrixes from the synthetic media.

The product of nanomatrixes can be concentrated, separated or purified from the buffer by numerous methods, such as dialysis, centrifugation, ultra-filtration, adsorption to solid surfaces, electrodialysis, or chromatography. They can also be kept as a dry powder after lyophilization of the suspension, to be reconstituted for later use with a suitable medium.

In another aspect the invention provides a composition for use in therapy, said composition further comprising a biologically interactive ingredient. In the product of the present invention, preferably at least one biologically interactive ingredient is either covalently pre-bonded to the protein, covalently bonded to the protein during cross-linking of nanomatrix, or trapped within the nanomatrixes or absorbed on the surface of the protein.

Further preferred embodiments are defined in the dependent claims.

The microscopic particles produced by the novel and unique method of the present invention are generally uniform in size which is typically less than 1 micron in diameter, and are porous. Therefore these microscopic particles are nanomatrixes.

It has also been discovered, that when they are properly produced by the novel and unique method of the present invention, the nanomatrixes are completely monodispersed with no aggregates and very stable in the synthetic media for a substantial length of time.

It has further been discovered, that pure hemoglobin (Hb) nanomatrixes could be synthesized in a normal saline buffer but they are unstable and tend to aggregate together. Presence of human serum albumin (HSA) of more than 4% (weight of HSA per total weight of protein) in the initial solution will provide stability to the primarily Hb nanomatrixes (about 96% by weight of total protein mass). For example a composition of essentially 96±2% hemoglobin (Hb) and 4±2% human serum albumin (HSA) can be used. A higher HSA to hemoglobin ratio also resulted to form progressively smaller nanomatrixes.

It has further been discovered that pure Hb nanomatrixes could also be synthesized in water but they are unstable and tend to aggregate together. Presence of at least one of stabilizer such as sodium lauryl sulphate (SLS) or Gelatin will provide stability to the primarily Hb nanomatrixes.

It has further been discovered that a higher gelatin to hemoglobin ratio resulted in smaller nanomatrixes. No significant difference in size was observed by using a higher HSA to hemoglobin ratio on nanomatrixes synthesized in butanol, but a difference in size was observed by using a higher HSA to hemoglobin ratio on nanomatrixes synthesized in propanol.

It has further been discovered that higher concentrations of hemoglobin solution resulted in larger nanomatrixes, and at least 4.76 percent by weight of gelatin is needed for preventing aggregates occurring in synthesis of Hb nanomatrixes.

It has further been discovered that the absence of a stabilizer such as hemoglobin from HSA solution resulted in unstabilized synthesis or useless aggregates. However, inclusion of as little as 1.48% of hemoglobin will greatly increase the stability of the HSA nanomatrixes.

It has further been discovered that a substantially high concentration of surfactant resulted in larger HSA nanomatrix size; excessive surfactant even resulted aggregates. When a critical amount of neutral organic solvent was exceeded by a small amount, the result was aggregated spheres instead of monodispersed nanomatrixes. The highest final concentration of non-dialyzed HSA (before addition of neutral organic solvent) that can produce monodispersed nanomatrixes in the absence of Hb is 148 mg/ml. With dialyzed HSA the final concentration that can be used to produce monodispersed HSA nanomatrixes without Hb may be higher. Also, methanol tends to produce smaller spheres than ethanol. It may be possible that a low STS concentration such as 1.4 mg/ml in conjunction with methanol would produce useful nanomatrixes with final concentrations of dialyzed HSA even higher than 148 mg/ml. Different neutral organic solvents have different effects on the sizes of Hb and HSA nanomatrixes.

It has further been discovered that drugs or biologically effective molecules trapped by or adhered to Hb and HSA nanomatrixes during encapsulation or thereafter, are stable and effective. Also, the biological agents pre-bonded to hemoglobin molecules can be incorporated into Hb containing nanomatrixes.

It is also anticipated that the uses of a cross-linking agent in conjunction with the method of the present invention will substantially decrease the reversibility of the nanomatrixes.

Further novel features and other objects of the present invention will become apparent from the following detailed description, discussion and the appended claims.

The present invention differs from all prior arts in that no cross-linking agents or heat denaturation procedures are necessary for the synthesis of the nanomatrixes. All the prior art employ these undesirable methods because without them the microspheres will not form or will fall apart immediately upon the slightest perturbation of the synthetic media. It is a surprise finding that stable nanomatrixes can be formed without cross-linking agents or heat denaturation procedures. The present invention results in nanomatrixes which are stable in the synthetic media, and yet are easily reversible within a reasonable time after injection into a host.

The present invention also optionally includes addition of various amounts of cross-linking agent as an optional step to increase the stability of the product to a mild and variable extent. The amount of cross-linking agent used should be less than saturation for all the available sites on the proteins so that no excessive cross-linking agent need to be neutralized or removed.

The present invention also allows microscopic particles, typically less than 1 micrometer in diameter, completely monodispersed with no aggregates, to be formed under ambient conditions by simple mixing of stable ingredient solutions. There are many advantages to such a method. Stable synthesis material can be stored separately and mixed only immediately before the nanomatrixes are to be synthesized. No elaborate machines are needed. All the ingredients are biocompatible and when given in appropriate concentration, are not harmful to the host. Expensive separation technologies to remove toxic material are not required because the present invention do not require their employment.

These particles are called nanomatrixes because they are typically less than 1 micrometer in diameter and are porous, which allows them to serve as scaffolds or hollow matrixes for information or other biologically active molecules to bind to or trapped therein. The porous nature of the structure allows the substrate of biologically active molecules to diffuse into the interior of the nanomatrix and for reaction products to diffuse out. Similarly, drugs can diffuse out of the nanomatrixes at rates dependent on the porosity of the nanomatrix carrier. Nanomatrixes are expected to be stable in the synthetic media.

In synthetic steps where no cross-linking process or agents were used, it was initially expected that the nanomatrixes formed would easily be reversible upon dilution of the ingredient solutions. It is therefore a surprising finding that certain types of nanomatrixes can be formed which are stable for a substantial length of time in different buffer solutions. The mechanism for prolonged stability of the nanomatrixes is not known. It is possible that upon assembly of the correct proportion of different molecules, certain hydrophobic regions are built up such that a greater stability is achieved with the nanomatrix than is possible with or anticipated from the individual molecules. An analogy would be the stability of an assembled jigsaw puzzle where individual pieces are interlocked by their matching borders to generate stability for the whole puzzle.

Other nanomatrixes have a shorter time of stability when the composition of the synthetic media is changed by either removal of some agents or a change in osmolarity of the synthetic media.

Ideally, the nanomatrixes are formed from biocompatible proteins with low antigenicity. One such protein is human serum albumin (HSA) which is the body's natural material for binding drugs. Nanomatrixes synthesized from HSA can provide a scaffold or a porous structure onto which the drugs can be absorbed or trapped. After injection, the nanomatrix may dissolve inside the blood stream. The drugs will equilibrate between the HSA originating from the nanomatrixes and the patient's own endogenous serum albumin molecules. Alternatively, some other nanomatrixes will dissolve only after entry into a cell, with the biological agent released thereafter.

Another naturally occurring molecule of low antigenicity is hemoglobin (Hb). In addition to providing a porous structure for drug adsorption, hemoglobin molecules can bind oxygen which is the drug of choice for many pathological conditions related to ischemia, such as coronary obstruction, cerebral vascular constriction, pulmonary vascular constriction, peripheral vascular disease and sickle cell crisis. Hemoglobin nanomatrixes can be made to carry bloodclot-dissolving agents such as urokinase or streptokinase. Injection of such dual function nanomatrixes into patients suffering from acute coronary spasm, thrombosis or embolism can have the advantage that some nanomatrixes (being about 0.1 micron small) will be able to navigate through the partial occlusion (which are probably several orders of magnitude larger in diameter) to deliver oxygen to the distal sites while other nanomatrixes retained by the blood clot will work on dissolving the clot to recannulate the vessel.

Another biological molecule of low allergic potential (hypoallergic) or low antigenicity is gelatin. Gelatin is described in The Merck Index (10th Ed.) as a heterogenous mixture of water-soluble hydrophilic proteins of high average molecular weight, derived from collagen by hydrolytic action, either by acidic or by alkaline hydrolysis. Gelatin has reactive surface groups that allow chemical modification or co-valent bonding with other biologically interactive ingredients before incorporation of such modified or biologically-enhanced molecules into the matrix of nanomatrixes.

Stringent requirements are imposed on the synthesis of reversible capsules and listed as follows:
a. The nanomatrixes synthesized must be uniform in size and small enough so that they do not obstruct even the capillaries which are about 7 microns in diameter. Ideally, the nanomatrixes should be less than 1 micron in diameter, hence the name nanomatrixes.
b. All the material used for synthesis of the nanomatrixes must be biocompatible and biodegradable. Conventional methods using high salt concentrations (such as 20% sodium sulphate) are themselves irritable to the veins and hyperosmotic and therefore not suitable for the purpose.
c. The method should produce nanomatrixes within a few minutes, ideally within seconds of mixing of reagents.
d. No complicated instrumentation is needed. All that is required is simple mixing of material pre-contained in two separate compartments in a binary container.
e. The reagents before mixing should have long, stable shelf-lives and can withstand extremes of temperatures and light during storage.
f. The nanomatrixes formed from mixing of reagents (by removal of the barrier between the two compartments in the binary container) must be stable within the synthetic media such that no aggregation would occur if the medication is not immediately administered to the patient.
g. The nanomatrixes must stay as a homogenous suspension of particles without formation of sediments within the synthetic media.
h. The synthetic media should be capable of further dilution with a suitable diluent without adversely affecting the nanomatrixes, for the purpose of adjusting for the dosage or final concentration of the drug, or osmolarity of the buffer as needed by the patient.
i. The nanomatrixes must be stable within the blood stream for a reasonable time before dissolving into its components.
j. The manufacturing process should not introduce new antigenicity to the protein molecules forming the nanomatrix. In the absence of any covalent-bond forming cross-linking agents, or extreme conditions of freezing or heating, the protein molecules are expected to escape denaturation or other conformational changes that can create or expose new antigenic sites.

Extensive amount of experiments have been performed in great detail for the study and research of the present invention. The following experiments have been performed to prove the validity of the present invention.

In the following experiments, stock hemoglobin (Hb) solutions were crystalline stroma-free human hemoglobin solution (approximately 60 mg/ml) maintained in a buffer containing at least 1.9 millimolar of potassium, 17 millimolar of sodium and 0.17% (w/v) of glucose; human serum albumin, (HSA), were purchased from pharmaceutical companies, typically as 250 mg/ml solutions in normal saline; other reagents were typically dissolved and diluted in water unless otherwise specified, e.g. surfactant, sodium lauryl sulphate (SLS) and sodium tetradecyl sulphate (STS). It is anticipated that other suitable cationic, anionic and non-ionic surfactants may also be used.

Although hemoglobin is used in these experiments, a variety of other globin proteins can be used, such as methemoglobin, polymerized hemoglobin, internally cross-linked hemoglobin, hemoglobin subunits, pyridoxylated hemoglobin, myoglobin and recombinant hemoglobins.

Similarly, although human serum albumin is mainly used in these experiments, a number of other hydrophilic proteins can be used such as immunoglobulins, albumin produced by recombinant DNA techniques, and albumin from other animal sources.

### EXPERIMENT ONE

### TITLE Synthesis of hemoglobin (Hb) nanomatrixes in normal saline buffer with human serum albumin (HSA) as stabilizer.

### EXPERIMENT

Various amount of a standard concentration of reagents or a fixed amount of reagent of various concentrations were mixed in one test tube in room temperature as listed in Table 1. Then 0.75 ml isopropanol (PROH) from a second test tube was then added quickly to the first test tube (tube 1). Within 10 seconds of vigorous mixing of the contents of the two test tubes, turbidity appeared. Examination of the content of the suspension under phase contrast microscopy revealed monodispersed nanomatrixes in tube 1 to 5 with visually homogenous sizes, as listed in Table 1. No aggregates were seen except in tube 6, 7, 8. The suspensions in tube 1 to 5 was stable in room temperature without aggregation for at least 4 days. No sedimentation or aggregation of nanomatrixes were observed during the four days in tube 1 to 5. In contrast, sediments were observed in tube 6, 7, 8 within hours of synthesis.

Aliquots of the nanomatrix preparations were placed in separate dialysis bags and dialyzed against at least 100 fold in volume of normal saline (NS) to check the stability of nanomatrixes upon removal of the neutral organic solvent propanol (PROH). Surprisingly, the nanomatrixes synthesized in tube 1 to 5 remained intact without aggregation or dissolution even after four days of dialysis. In contrast, large aggregates were seen inside the dialysis bags containing material synthesized in tube 6 to 8 within one hour of synthesis.

### CONCLUSION

Pure hemoglobin nanomatrixes (without presence of HSA) could be synthesized in a normal saline buffer but they are unstable and tend to aggregate together. Presence of more than 4% HSA (weight of HSA per total weight of protein) in the initial solution will provide stability to the primarily hemoglobin nanomatrixes (about 96% of total protein mass, assuming an equal ratio of Hb to HSA molecules are incorporated into the nanomatrixes). A higher HSA to hemoglobin ratio also resulted to form progressively smaller nanomatrixes.

**TABLE 1**

| Tube | Hb 60mg/ml | HSA 0.1 ml | NS | PROH | SIZE micron |
|---|---|---|---|---|---|
| 1 | 0.225ml | 200mg/ml | 2.675ml | 0.75ml | 0.8 |
| 2 | 0.225ml | 100mg/ml | 2.675ml | 0.75ml | 1.0 |
| 3 | 0.225ml | 50mg/ml | 2.675ml | 0.75ml | 1.2 |
| 4 | 0.225ml | 25mg/ml | 2.675ml | 0.75ml | 1.5 |
| 5 | 0.225ml | 12mg/ml | 2.675ml | 0.75ml | 1.5 |
| 6 | 0.225ml | 6mg/ml | 2.675ml | 0.75ml | 2 to 4 |
| 7 | 0.225ml | 3mg/ml | 2.675ml | 0.75ml | 2 to 4 |
| 8 | 0.225ml | 0mg/ml | 2.675ml | 0.75ml | 2 to 4 |

### EXPERIMENT TWO

### TITLE Synthesis of hemoglobin (Hb) nanomatrixes in low ionic strength buffer with sodium tetradecyl sulphate (STS) or gelatin as stabilizer.

### EXPERIMENT

Sotradecol 3% is a brand of sodium tetradecyl sulfate (STS) approved for intravenous injection by the Food and Drug Administration (FDA). At the 3% concentration, STS was irritable to veins. However, lower concentrations are acceptable. Gelatin solutions were prepared by dissolving gelatins in distilled water (2% w/v).

Reagents (hemoglobin stock solution, water, gelatin or STS) were mixed in first test tube (e.g., tube 9) according to Table 2. Subsequently aliquots of alcohols, such as propanol (PROH), butanol (BUOH) or ethanol (ETOH), from a second test tube were added quickly to the contents of first test tube. Turbidity appeared within ten seconds and homogenous monodispersed nanomatrixes (typically less than 0.2 micron in diameter) were seen under phase microscopy in tube 9 to 17. These suspensions were homogenous and stable for at least 8 days without formation of sediments or aggregations.

In contrast, large aggregates were seen with the unaided eye in tube 18, 19, 20 demonstrating that at least one stabilizer (either STS or gelatin molecules) must be present for stable monodispersed nanomatrixes to form.

Aliquots of the contents of tube 9 to 20 were serially diluted (2-fold steps) in either distilled water or 5% HSA solution in normal saline (to simulate the intravascular environment) until the original suspension became at least 1000 less concentrated. After at least 1000 fold dilution in either water or 5% HSA solution in normal saline, the contents from all tubes (9 to 20) still appeared turbid, indicating that the nanomatrixes (whether monodispersed or in aggregates) were stable to dilution.

### CONCLUSION

Pure hemoglobin nanomatrixes (without presence of HSA) could also be synthesized in water or hypotonic solutions but they are unstable and tend to aggregate together. Presence of at least one of the stabilizers (STS or Gelatin) will provide stability to the primarily hemoglobin nanomatrixes.

**TABLE 2**

| Tube | Hb 60mg/ml | WATER | STS 2.8mg/ml | GEL 20mg/ml | ALCOHOL | | |
|---|---|---|---|---|---|---|---|
| | | | | | PROH | BUOH | ETOH |
| 9 | 0.225ml | 2.525ml | 0.25ml | none | none | none | 1.10ml |
| 10 | 0.225ml | 2.525ml | 0.25ml | none | none | 0.20ml | none |
| 11 | 0.225ml | 2.525ml | 0.25ml | none | 1.00ml | none | none |
| 12 | 0.225ml | 2.675ml | none | 0.1ml | none | none | 1.25ml |
| 13 | 0.225ml | 2.675ml | none | 0.1ml | none | 0.20ml | none |
| 14 | 0.225ml | 2.675ml | none | 0.1ml | 0.75ml | none | none |
| 15 | 0.225ml | 2.425ml | 0.25ml | 0.1ml | none | none | 1.23ml |
| 16 | 0.225ml | 2.425ml | 0.25ml | 0.1ml | none | 0.20ml | none |
| 17 | 0.225ml | 2.425ml | 0.25ml | 0.1ml | 1.00ml | none | none |
| 18 | 0.225ml | 2.775ml | none | none | none | none | 1.20ml |
| 19 | 0.225ml | 2.775ml | none | none | none | 0.20ml | none |
| 20 | 0.225ml | 2.775ml | none | none | 0.75ml | none | none |

### EXPERIMENT THREE

### TITLE Effect of a higher stabilizer molecule to hemoglobin (Hb) ratio on synthesis of nanomatrixes.

### EXPERIMENT

Reagents were again mixed in first test tube (e.g., tube 21) as listed in Table 3 and subsequently 0.3 ml of butanol (BUOH) was added from second test tube to produce turbidity.

The sizes of nanomatrixes formed are also shown in Table 3. The nanomatrixes were homogenous in size for each preparation and stable to dilution to over 100x in distilled water.

### CONCLUSION

A higher gelatin to hemoglobin ratio resulted in smaller nanomatrixes. However, no significant difference in size was observed by using a higher HSA to hemoglobin ratio on nanomatrixes synthesized in butanol (BUOH), as compared to the result from Experiment One as shown in Table 1, where a higher HSA to hemoglobin ratio resulted in progressively smaller nanomatrixes (tube 1 to 5) when formed in propanol (PROH).

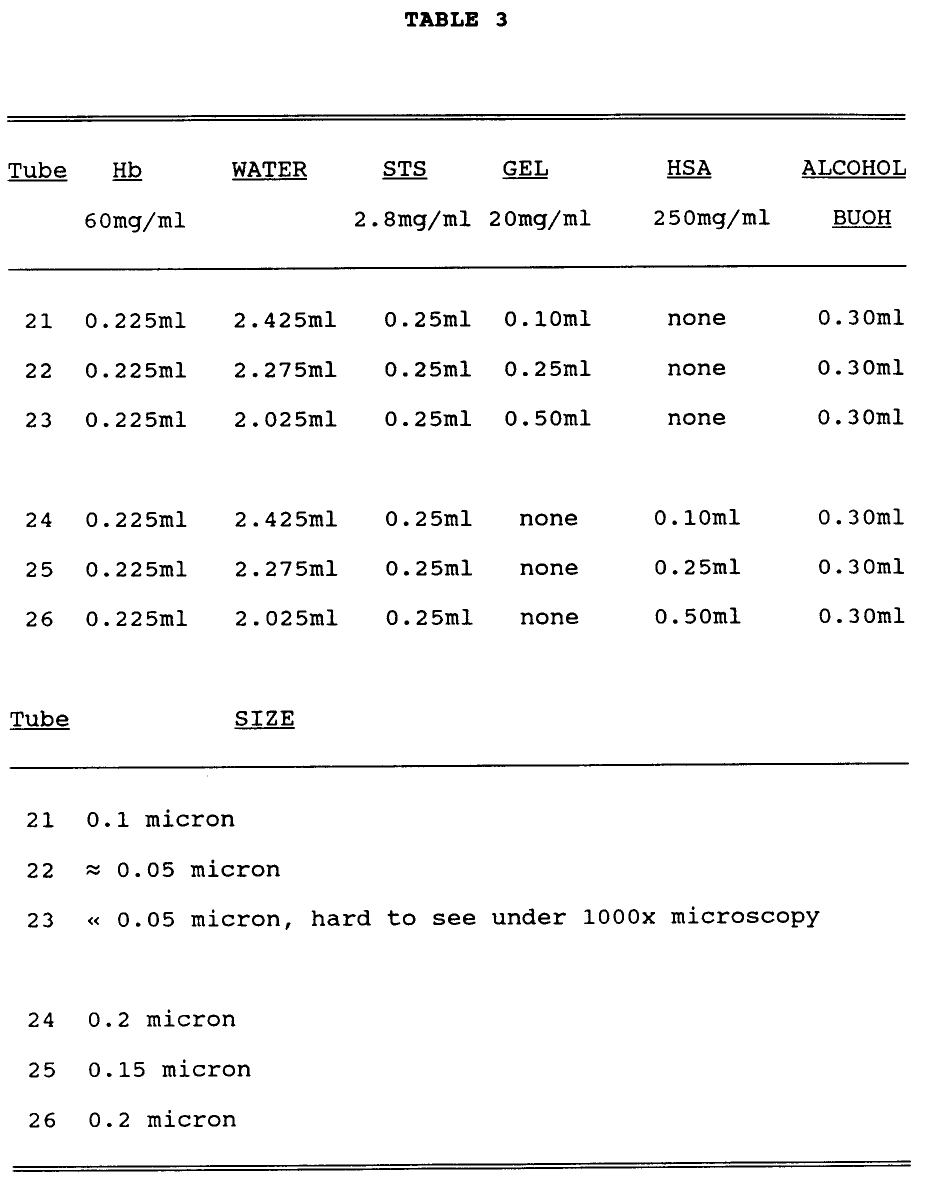

### EXPERIMENT FOUR

### TITLE Effect of a higher concentration of hemoglobin (Hb) on synthesis of nanomatrixes.

### EXPERIMENT

The concentration of hemoglobin molecules has been increased in tube 27-29, as shown in Table 4. As a result, monodispersed nanomatrixes were observed with average size of 1.0 to 1.2 microns in diameter.

### CONCLUSION

Higher concentrations of hemoglobin solution resulted in larger nanomatrixes.

**TABLE 4**

| Tube | Hb 60mg/ml | WATER | STS 2.8mg/ml | HSA 250mg/ml | ALCOHOL BUOH |
|---|---|---|---|---|---|
| 27 | 2.25ml | 0.40ml | 0.25ml | 0.10ml | 0.30ml |
| 28 | 2.25ml | 0.25ml | 0.25ml | 0.25ml | 0.30ml |
| 29 | 2.25ml | 0.00ml | 0.25ml | 0.50ml | 0.30ml |

### EXPERIMENT FIVE

### TITLE Synthesis of human serum albumin (HSA) nanomatrixes.

### EXPERIMENT

Stock HSA solutions (25% in normal saline) were diluted in distilled water to 80 mg/ml. Aliquots of this diluted HSA was mixed with various amounts of sodium lauryl sulphate (SLS) and distilled water as listed in Table 5. Subsequently 0.8 ml of ethanol (ETOH) was added. Only the minimal amount that is needed to produce turbidity is used. Excessive amounts of alcohol will lead to aggregation of nanomatrixes. Upon dilution with distilled water or normal saline buffer (1 vol to 1 vol), the contents of tubes 30 and 31 redissolved into a clear solution within one hour. It was also observed that aggregates had occurred in tube 32.

### CONCLUSION

Absence of a stabilizer such as hemoglobin from HSA solution resulted in nanomatrixes that can easily redissolve on dilution of the neutral organic solvent ETOH, such as occurred in tubes 30 and 31. Absence of a suitable surfactant such as SLS results in useless aggregates, such as occurred in tube 32.

**TABLE 5**

| Tube | HSA 80mg/ml | WATER | SLS 8mg/ml | ETOH | SIZE |
|---|---|---|---|---|---|
| 30 | 0.25ml | 0.500ml | 0.250ml | 0.8ml | 0.1µm |
| 31 | 0.25ml | 0.625ml | 0.125ml | 0.8ml | 0.05µm |
| 32 | 0.25ml | 0.750ml | 0.000ml | 0.8ml | aggr. |

### EXPERIMENT SIX

### TITLE Reversibility of human serum albumin (HSA) nanomatrixes synthesized in the presence of small amount of hemoglobin (Hb) as stabilizer.

### EXPERIMENT

Stock hemoglobin solutions were diluted to 3 mg/ml with distilled water and added to first test tube in the presence of STS (e.g., tube 33) as indicated in Table 6. Subsequently, a minimal amount of ethanol (ETOH) was used to cause turbidity. The turbid suspension was added into dialysis bags to be dialyzed against at least 100 fold of normal saline to remove the reagents including ethanol. Only tube 33 turned clear within 2 hours. The contents of tube 34 to 37 remain turbid for 3 more days of dialysis.

### CONCLUSION

Inclusion of as little as 1.48% (0.3 divided by 20 + 0.3 mg total protein) of hemoglobin in the initial mixture will greatly increase the stability of the HSA nanomatrixes.

**TABLE 6**

| Tube | HSA 80mg/ml | Hb 3mg/ml | WATER | STS 8mg/ml | ETOH |
|---|---|---|---|---|---|
| 33 | 0.25ml | 0.0ml | 0.5ml | 0.25ml | 0.8ml |
| 34 | 0.25ml | 0.1ml | 0.4ml | 0.25ml | 0.8ml |
| 35 | 0.25ml | 0.2ml | 0.3ml | 0.25ml | 0.4ml |
| 36 | 0.25ml | 0.3ml | 0.2ml | 0.25ml | 0.3ml |
| 37 | 0.25ml | 0.4ml | 0.1ml | 0.25ml | 0.2ml |

### EXPERIMENT SEVEN

### TITLE Encapsulation of drugs with human serum albumin (HSA) nanomatrixes.

### EXPERIMENT

Doxorubicin (ADR) was purchase from Adria and reconstituted with water to a final concentration of 0.3 mg/ml. A stable solution was prepared by mixing 0.10 ml or 0.25 ml of HSA (at 80 mg/ml, diluted with water) with various volumes of STS (8 mg/ml with water) and 0.25ml of doxorubicin solution (0.3 mg/ml with water), with or without hemoglobin (Hb). Subsequently, an aliquot of ethanol (ETOH) or propanol (PROH) was added with rapid shaking to mix them well. Red nanomatrixes were observed in preparations without hemoglobin (tube 103, 104, 105, 106) which redissolves within one hour to a clear red solution (color of ADR solutions) after dilution with an equal part of distilled water or normal saline buffer. Red nanomatrixes prepared with hemoglobin with an adequate amount of HSA (tube 101, 102) stayed intact after dilution with an equal part of distilled water or normal saline buffer for up to at least 24 hours.

### CONCLUSION

Hb and HSA nanomatrixes containing encapsulated drugs are stable with various degrees of reversibility. Although ADR is used here, other biological active molecules can similarly be incorporated either as a single entity or in combinations, such as receptor analogs (e.g., recombinant CD₄), receptor agonist or antagonist, inhibitors, stimulants, growth factors, hormones, cytokines, glycosylated proteins, amino acid, nucleic acid, carbohydrates, RNA, DNA, anti-sense polynucleotides, polypeptides, polymerases, viruses, lipids, steroids and ribozymes. Biologically interactive components, which may be by themselves inert but useful to biological systems, such as contrast dye and air bubbles, may also be incorporated by this method.

**TABLE 7**

| Tube | Hb 63mg/ml | HSA 80mg/ml | STS 8mg/ml | WATER | ADR 0.3mg/ml | ALCOHOL |
|---|---|---|---|---|---|---|
| | | | | | | PROH |
| 101 | 0.25ml | 0.1ml | 0.1ml | 0.15ml | 0.25ml | 0.20ml |
| 102 | 0.25ml | 0.1ml | 0.0ml | 0.25ml | 0.25ml | 0.35ml |

| | | | | | | ETOH |
|---|---|---|---|---|---|---|
| 103 | none | 0.25ml | 0.25ml | 0.25ml | 0.25ml | 0.80ml |
| 104 | none | 0.25ml | 0.20ml | 0.30ml | 0.25ml | 0.80ml |
| 105 | none | 0.25ml | 0.15ml | 0.35ml | 0.25ml | 0.80ml |
| 106 | none | 0.25ml | 0.10ml | 0.40ml | 0.25ml | 0.80ml |

### EXPERIMENT EIGHT

### TITLE Preparation of radioactive hemoglobin (Hb) nanomatrixes with alkaline phosphatase (AK) enzyme activity.

### EXPERIMENT

The purpose of the experiment was to find out if biological agents pre-bonded to protein molecules can be incorporated into Hb nanomatrixes. Radioactive iodine was chosen because its activity could easily be measured with a gamma counter.

An additional enzyme, alkaline phosphatase (AK), was added to the mixture to see if it could be incorporated into the nanomatrix and if so, whether enzymatic activity was preserved. The activity of AK could easily be measured and would serve as a model for other drugs such as enzymes (e.g., urokinase) or protein antibiotics (polymyxin), or biological molecules such as antibodies, growth factors, hormones (e.g. insulin) and cytokines (e.g., interleukins, interferons, tumor necrosis factor, prostaglandin, granulocyte-macrophage colony-stimulating factor).

An aliquot of hemoglobin solution was first iodinated with Iodine 125 by using standard chloramine-T linking method. The specific activity of the labeled hemoglobin solution was found to be approximately 6 microcurie per mg protein. The conditions of tube 2 in Experiment One (shown in Table 1) was used except that radioactive hemoglobin molecules were premixed with the nonradioactive hemoglobin solution to result in a final concentration of 60 mg/ml of radioactive hemoglobin of which 0.225 ml was used for synthesis of the nanomatrixes. Instead of normal saline buffer, a solution of alkaline phosphatase (0.1 mg/ml in normal saline buffer) was used.

After formation of nanomatrixes with the addition of 0.8 ml propanol (PROH), an aliquot was used to measure its radioactivity and AK activity. Another aliquot was centrifuged to remove all the nanomatrixes. The clear supernatant was subsequently assayed for its radioactivity and AK activity. It was found that at least 80 percent of the total radioactivity as well as AK activity resided with the nanomatrix.

### CONCLUSION

The biological agents pre-bonded to hemoglobin molecules can be incorporated into Hb nanomatrixes. Biologically active molecules can be trapped within the relatively stable nanomatrixes and retain their biological activities.

### EXPERIMENT NINE

### TITLE Lowest amount of gelatin needed as stabilizer for synthesis of hemoglobin (Hb) nanomatrixes in hyperosmolar buffer.

### EXPERIMENT

Reagents were mixed in first test tube (e.g., tube 38) as listed in Table 8. Buffer used contained 3x normal saline (i.e. 465 millimolar NaCl) with 20 millimolar sodium phosphate (pH 7.4), dissolved in water (total osmolarity equal to at least 970 milliosmos).

Radioactive hemoglobin (radiolabeled with iodine-125 purchased from commercial source) was added to stock nonradioactive hemoglobin solution to generate a mixture with radioactivity of about 5,000 cpm per mg hemoglobin.

After equilibration for at least 5 minutes, about 1.12 ml of butanol (BUOH) was added to each tube and the contents well shaken. Aliquots from the resultant turbid suspensions were examined under phase microscopy. All preparations from tube 38 to 42 contained monodisperse nanomatrixes about 0.05 micron in diameter. In contrast, tube 43 to 45 contained aggregates of nanomatrixes.

The percentage of radioactive hemoglobin molecules that were incorporated into the nanomatrixes were calculated as follows:
a. Radioactivity from aliquots of suspensions were counted in a gamma counter to obtain cpm per ml of suspension.
b. Nanomatrixes were removed by centrifugation in high speed for at least 20 minutes.
c. Radioactivity from respective aliquots of the clear supernatant were counted.
d. Differences between cpm per ml of whole suspension and that of the supernatant equals the cpm of nanomatrixes originally suspended in one ml of suspension.
e. The Percentage yield = cpm of nanomatrixes obtained from one ml of suspension divided by cpm of the one ml of original suspension.
f. The yield of nanomatrixes ranged from 73% to 95%.

Aliquots of the contents from tube 38 to 45 were placed in dialysis bags and dialyzed in over 100 fold normal saline to test the integrity of the nanomatrixes. It was noticed that tube 38 to 42 had no aggregation for at least four days, while aggregates were visible to the unaided eye for contents from tube 43 to 45 within one hour of dialysis.

Dilution of one volume of the aliquots from tube 38 to 45 with two volumes of water to produce a final concentration of sodium chloride equivalent to normal saline buffer showed that the nanomatrixes produced in tube 38 to 45 were all stable in normal saline buffer except that those from tube 38 to 42 remained as monodispersed nanomatrixes and those from 43 to 45 remained aggregated.

### CONCLUSION

More than 3.6 percent by weight of gelatin in the initial solution is needed for preventing aggregates occurring in synthesis of Hb nanomatrixes. Assuming equal proportions of Hb and gelatin are incorporated into nanomatrixes, this experiment suggests that more than 3.6% of total weight of protein should be gelatin to maintain monodispersity of primarily Hb nanomatrixes.

**TABLE 8**

| Tube | Hb 50mg/ml | BUFFER | STS 3.2mg/ml | GEL 10mg/ml | BUOH |
|---|---|---|---|---|---|
| 38 | 0.80ml | 8.20ml | 1.0ml | 1.00ml | 1.12ml |
| 39 | 0.80ml | 8.40ml | 1.0ml | 0.80ml | 1.12ml |
| 40 | 0.80ml | 8.60ml | 1.0ml | 0.60ml | 1.12ml |
| 41 | 0.80ml | 8.80ml | 1.0ml | 0.40ml | 1.12ml |
| 42 | 0.80ml | 9.00ml | 1.0ml | 0.20ml | 1.12ml |
| 43 | 0.80ml | 9.05ml | 1.0ml | 0.15ml | 1.12ml |
| 44 | 0.80ml | 9.10ml | 1.0ml | 0.10ml | 1.12ml |
| 45 | 0.80ml | 9.20ml | 1.0ml | 0.00ml | 1.12ml |

### EXPERIMENT TEN

### TITLE Effect of surfactant concentration on synthesis of human serum albumin (HSA) nanomatrixes.

### EXPERIMENT

As shown in Table 9, 2.5 ml of sodium lauryl sulphate (SLS) of various concentrations were mixed with 2.5 ml of HSA (60 mg/ml diluted in water). After 5 minutes of equilibration, 5.5 ml of ethanol (ETOH) was added to each tube.

Aliquots of the contents from tube 46 to 48 were examined under phase microscopy. It was noted that tube 46 and 47 contained monodispersed nanomatrixes whereas tube 48 contained useless aggregates consisting of thousands of spheres each 1.5 to 2 microns in diameter.

### CONCLUSION

Substantially high concentration of surfactant resulted in larger HSA nanomatrix size, even aggregates.

**TABLE 9**

| Tube | HSA 60mg/ml | SLS 2.5ml | ETOH | SIZE micron |
|---|---|---|---|---|
| 46 | 2.5ml | 6mg/ml | 5.5ml | 0.8 |
| 47 | 2.5ml | 10mg/ml | 5.5ml | 1.2 |
| 48 | 2.5ml | 14mg/ml | 5.5ml | aggregates of 1.5 to 2 micron spheres |

### EXPERIMENT ELEVEN

### TITLE Effect of the concentration of surfactant on synthesis of hemoglobin (Hb) nanomatrixes.

### EXPERIMENT

Hemoglobin solutions (30 mg/ml) were mixed with 0.1 ml of various concentrations of sodium lauryl sulphate (SLS) as shown in Table 10. Subsequently 0.35 ml of ethanol (ETOH) was added to the 1.1 ml of mixture and shaken to produce turbidity.

### CONCLUSION

Again the concentration of surfactant is important. Increasing concentrations lead to larger nanomatrixes, even aggregations.

**TABLE 10**

| Tube | Hb 30mg/ml | SLS 0.1ml | ETOH | SIZE micron |
|---|---|---|---|---|
| 49 | 1.0ml | 7mg/ml | 0.35ml | 0.1 |
| 50 | 1.0ml | 9mg/ml | 0.35ml | 0.2 |
| 51 | 1.0ml | 11mg/ml | 0.35ml | aggregates of 0.3 micron spheres |

### EXPERIMENT TWELVE

### TITLE Effect of increasing amounts of neutral organic solvent on synthesis of hemoglobin (Hb) and human serum albumin (HSA) nanomatrixes.

### EXPERIMENT

Reagents were mixed in tubes (e.g., tube 52) as listed in Table 11. Subsequently, 0.50 to 0.66 ml of ETOH was added to the tubes.

### CONCLUSION

When a critical amount of neutral organic solvent was exceeded by a small amount, the result was aggregated spheres instead of monodispersed nanomatrixes.

**TABLE 11**

| Tube | Hb 63mg/ml | HSA 250mg/ml | SLS 9mg/ml | ETOH | SIZE micron |
|---|---|---|---|---|---|
| 52 | 1ml | 0.2ml | 0.12ml | 0.50ml | < 0.1 |
| 53 | 1ml | 0.2ml | 0.12ml | 0.55ml | 0.1 |
| 54 | 1ml | 0.2ml | 0.12ml | 0.58ml | 0.1 |
| 55 | 1ml | 0.2ml | 0.12ml | 0.62ml | 0.2 |
| 56 | 1ml | 0.2ml | 0.12ml | 0.66ml | small aggregates |

### EXPERIMENT THIRTEEN

### TITLE Effect of osmolarity (hypotonicity) on synthesis of human serum albumin (HSA) nanomatrixes.

### EXPERIMENT

Standard HSA solutions with HSA dissolved in normal saline buffer were diluted with water and mixed with an equal volume of a suitable concentration of surfactant. After equilibration, a minimum amount of methanol was added quickly with shaking of the tube to produce turbidity. Non-dialyzed standard HSA solutions were used with Ethanol as well.

Dialyzed HSA were previously dialyzed extensively with distilled water before an equal volume of surfactant was added. After equilibration, a minimum amount of methanol was added with shaking to produced turbidity.

Ethanol was also used on non-dialyzed as well as dialyzed HSA solutions to see if the effects of low osmolarity were applicable with ETOH as neutral organic solvent.

The results are displayed in the following tables:
Table 12: Using non-dialyzed HSA with Methanol.
Table 13: Using dialyzed HSA with Methanol.
Table 14: Using non-dialyzed HSA with Ethanol.
Table 15: Using dialyzed HSA with Ethanol.

In all the tables 12-15, the column labels appearing in the top horizontal list are the concentrations of STS (mg/ml, dissolved in water) immediately before addition of neutral organic solvent, and the row labels appearing in the left vertical list are concentrations of HSA (mg/ml) immediately before the addition of neutral organic solvent.

### CONCLUSION

These experiments are designed in such a way that upon dilution of the non-dialyzed stock HSA solution with distilled water, the concentration of HSA as well as sodium chloride-containing solvent were equally and proportionally diluted. In Table 12, as the concentration of HSA reached down to 46 mg/ml, the sodium concentration was approximately (150 X 46/250) or 27.6 milliequivalent. Even at such a low osmolarity, no useful nanomatrixes were formed with the wide range of STS concentration used in conjunction with methanol as neutral organic solvent. In contrast, by using HSA extensively dialyzed in water to remove practically all free sodium ions, useful nanomatrixes were obtained (Table 13) at HSA concentration as high as 110 mg/ml in conjunction with STS concentration of 7.7 mg/ml. Again, the adverse effect of higher concentration of STS than optimal was observed. Similarly dialyzed HSA in conjunction with STS at concentration of 11.5 mg/ml produced useless aggregates.

When the experiments were repeated using ethanol as neutral organic solvent, the highest concentration of HSA that could result in monodispersed nanomatrixes was 148 mg/ml, in conjunction with STS concentration as low as 1.4 mg/ml (Table 14). Again, higher than optimal concentration of STS (e.g., 7.4 mg/ml) resulted in useless aggregates.

Attempts to repeat the effects of high concentrations of dialyzed HSA was hampered by the fact that the stock HSA (non-dialyzed) solution became inevitably diluted with water during dialysis. Table 15 showed, however, that nanomatrixes of extremely small sizes could be obtained from dialyzed HSA up to the concentration of 110 mg/ml in conjunction with STS concentration of 2.3 mg/ml. Too low (0.8 mg/ml) or too high (e.g., 7.4 mg/ml) concentration of STS resulted in useless aggregates. Concentration of dialyzed HSA above 148 mg.ml (if available) will probably produce useful nanomatrixes also.

These experiments showed the complexity of various conditions that must be within narrow ranges for useful nanomatrix production. For example, low ionic strength (e.g., dialyzed HSA) allows a higher concentration of HSA to be used (compare Table 12 with 13). However, STS concentrations must be within a narrow range. The effect of neutral organic solvent was also obvious: using non-dialyzed HSA, methanol produced no useful nanomatrixes at STS concentration as low as 2.7 mg/ml and HSA concentration as low as 74 mg/ml (Table 12). However, ethanol produced useful nanomatrixes at HSA concentrations up to 148 mg/ml in conjunction with STS of 1.4 mg/ml (Table 14). In general methanol produced nanomatrixes smaller than ethanol.

**TABLE 12**

| STS (mg/ml in water) | | | | | | | |
|---|---|---|---|---|---|---|---|
| | 11.5 | 7.3 | 6.4 | 5.5 | 4.5 | 3.6 | 2.7 |
| HSA (mg/ml) | | | | | | | |
| 110 | X | X | X | X | X | X | X |
| 102 | X | | | | | | |
| 92 | X | X | X | X | X | X | X |
| 83 | X | | | | | | |
| 74 | X | X | X | X | X | X | X |
| 64 | X | | | | | | |
| 55 | X | | | | | | |
| 46 | X | | | | | | |
| X = Useless aggregates | | | | | | | |

**TABLE 13**

| STS (mg/ml in water) | | | | | | | | | | |
|---|---|---|---|---|---|---|---|---|---|---|
| | 11.5 | 9.1 | 8.2 | 7.7 | 7.3 | 6.4 | 5.5 | 4.5 | 3.6 | 2.7 |
| HSA (mg/ml) | | | | | | | | | | |
| 110 | X | | | C | | | | | | |
| 102 | X | | | C | | | | | | |
| 92 | X | | | X | | | | | | |
| 83 | X | | | D | | | | | | |
| 74 | X | X | X | X | X | D | X | D | D | D |
| 64 | X | | | C | | | | | | |
| 55 | X | | | C | | | | | | |
| 46 | X | | | C | | | | | | |
| C = Less than 0.05 micron spheres D = Less than 0.1 micron spheres X = Useless aggregates | | | | | | | | | | |

**TABLE 14**

| STS (mg/ml in water) | | | | | | | | | | |
|---|---|---|---|---|---|---|---|---|---|---|
| 24.5 | 18.2 | 13.6 | 9.1 | 7.4 | 5.5 | 3.6 | 1.8 | 1.4 | 0.9 | 0 |
| HSA (mg/ml) | | | | | | | | | | |
| 227 X | X | X | X | X | X | X | X | X | X | X |
| 216 | | | | | | | | X | | |
| 204 | | | | | | | | X | | |
| 193 | | | | | | | | X | | |
| 185 | | | | X | | | | | | |
| 182 | | | | | | | | X | | |
| 170 | | | | | | | | X | | |
| 159 | | | | | | | | X | | |
| 148 | | | | | | | | B | | |
| 136 | | | | X | | | | B | | |
| 125 | | | | | | | | B | | |
| 113 | | | | | | | | B | | |
| 102 | | | | | | | | B | | |
| 91 | | | | X | | | | B | | |
| 79 | | | | | | | | B | | |
| 74 | | | | A | | | | | | |
| 56 | | | | A | | | | | | |
| 37 | | | | B | | | | | | |
| A = 0.5 to 8 micron spheres B = 1 to 2 micron spheres X = Useless aggregates | | | | | | | | | | |

**TABLE 15**

| STS (mg/ml in water) | | | | | | | | | | | |
|---|---|---|---|---|---|---|---|---|---|---|---|
| | 9.1 | 8.2 | 7.4 | 6.4 | 5.5 | 4.5 | 3.6 | 2.7 | 2.3 | 1.4 | 0.8 |
| HSA (mg/ml) | | | | | | | | | | | |
| 110 | | | | | | | | | H | F | X |
| 101 | | | | | | | | | E | E | X |
| 92 | | | | | | | | | G | H | X |
| 83 | | | | | | | | | G | H | X |
| 74 | X | X | X | E | F | F | G | G | E | E | X |
| 64 | | | | | | | | | I | H | X |
| E = 0.1 micron spheres F = 1 to 3 micron spheres G = 0.3 to 0.5 micron spheres H = Less than 0.05 micron spheres I = 0.8 to 1 micron spheres X = Useless aggregates | | | | | | | | | | | |

### EXPERIMENT FOURTEEN

### TITLE Effect of neutral organic solvent on synthesis of hemoglobin (Hb) and human serum albumin (HSA) nanomatrixes.

### EXPERIMENT

Reagents were mixed in first tube in accordance with Table 16. After equilibration, 0.15 ml of the respective alcohol was added and the tubes were immediately shaken to produce turbidity.

Examination of an aliquot of the content from tube 57, 58 and 59 showed monodispersed nanomatrixes of sizes 0.05, 0.5 and 1.5 micron respectively.

### CONCLUSION

Different neutral organic solvent have different effects on the sizes of Hb and HSA nanomatrixes.

**TABLE 16**

| Tube | Hb 60mg/ml | HSA 250mg/ml | STS 30mg/ml | ALCOHOL | | | SIZE micron |
|---|---|---|---|---|---|---|---|
| | | | | ETOH | PROH | BUOH | |
| 57 | 1.0ml | 0.12ml | 0.112ml | 0.462ml | none | none | 0.05 |
| 58 | 1.0ml | 0.12ml | 0.112ml | none | 0.462ml | none | 0.5 |
| 59 | 1.0ml | 0.12ml | 0.112ml | none | none | 0.462ml | 1.5 |

### EXPERIMENT FIFTEEN

### TITLE Synthesis of DNA-containing nanomatrixes.

### EXPERIMENT

Tube One contains 0.4 ml of a human serum albumin (250 mg/ml) mixed with 0.1 ml of a stroma free human hemoglobin solution (60 mg/ml, containing more than 10% as methemoglobin). To tube one was added 0.5 ml of a DNA solution (1.0 microgram dissolved in normal saline) with shaking to mix well. Ethyl alcohol (200 proof) was added dropwisely (approximately 0.4 ml) until turbidity appears. After the formation of nanomatrixes (examined to be 0.2 microns subsequently by microscopy), the entire suspension was divided equally into two tubes (tube 2, and tube 3). Tube 2 received 0.1 ml of a glutaraldehyde solution (0.01% v/v in normal saline), while tube 3 received 0.1 ml of normal saline as control solution. After another 5 minutes, both tube 2 and tube 3 received 3 ml of normal saline to dilute the nanomatrix suspension.

Assay of the DNA content in tube 2 and tube 3 both showed that more than 30% of the DNA were entrapped inside and on the surface of the nanomatrixes. After removal of the DNA in the supernatant, both DNA containing nanomatrixes were incubated in DNA-free media overnight. No DNA was found to leak out of the nanomatrixes. Attempts to degrade entrapped DNA released less than 20% of all the nanomatrix-bound DNA, suggesting that most of them were contained in the interior of the nanomatrixes. The similarity between the nanomatrixes in tube 2 and tube 3 suggested that addition of the cross-linking agent is purely optional and does not contribute to the formation of the nanomatrix itself.

### CONCLUSION

DNA can be successfully entrapped inside the nanomatrixes produced by the method of the present invention, and the use of cross-linking agent is purely optional for the present invention method.

### EXPERIMENT SIXTEEN

### TITLE Formation of RNA-containing nanomatrixes.

### EXPERIMENT

Experimental conditions were similar to Experiment 15 except the bovine hemoglobin solution was used instead of human hemoglobin solution (in the ratio of 1 volume of hemoglobin to 19 volumes of human serum albumin) and 0.5 microgram of RNA was used instead of the DNA added to tube 1. It was found that about 45% to 50% of the RNA were trapped during synthesis to the nanomatrixes. Leakage of RNA into the media was minimal.

### CONCLUSION

RNA can also be successfully entrapped inside the nanomatrixes produced by the method of the present invention, and the use of cross-linking agent is again purely optional for the present invention method.

### EXPERIMENT SEVENTEEN

### TITLE Attachment of DNA and RNA to the surface of preformed nanomatrixes.

### EXPERIMENT

Control nanomatrixes were synthesized in accordance with Experiment 15 tube 2 and tube 3 conditions except that no DNA was added (0.5 ml of normal saline was added to tube one). Ethanol was removed by dialysis overnight in 1000x excess of normal saline.

Subsequently, approximately 0.02 microgram of RNA was mixed with microliter of the preformed control nanomatrix suspensions, after which the nanomatrixes were separated from the supernatant by high speed centrifugation. Analysis of the RNA content indicated about 50% of the RNA became attached to the surface of the preformed nanomatrixes.

The experiment was repeated with addition of 0.1 microgram of DNA to 100 microliter of the preformed control nanomatrix suspensions. Analysis of the DNA content of the nanomatrixes indicated at least 20% of the DNA became attached to the preformed nanomatrixes.

### CONCLUSION

Both DNA and RNA can successfully attach to the surfaces of the nanomatrixes produced by the method of the present invention, and the use of cross-linking agent is again purely optional for the present invention method.

### EXPERIMENT EIGHTEEN

### TITLE Transformation of 3T3 cell line with DNA-containing nanomatrixes.

### EXPERIMENT

DNA carrying the gene for beta-galactosidase was encapsulated by two methods: (a) during synthesis of nanomatrixes using the method of tube 2 and tube 3 of Experiment 15; and (b) attachment to preformed nanomatrixes as in Experiment 17. Approximately an equivalence of 0.02 microgram of DNA encapsulated in nanomatrixes were added to cultured 3T3 cells. After incubation at 37 degree C for 2 to 4 days, substrate for the beta-galactosidase reaction was added to the cells. Clones of blue cells were seen among cultures to which nanomatrixes (regardless of method of synthesis and DNA entrapment) were added, which indicated successful expression of the foreign gene. Cultures exposed similarly to control nanomatrixes without DNA remained colorless. Cultures exposed to only DNA without nanomatrixes remained untransformed.

### CONCLUSION

The DNA-containing nanomatrixes produced by the method of the present invention can perform successful transformation of 3T3 cell line.

### EXPERIMENT NINETEEN

### TITLE Transformation of cells in live animals with DNA-containing nanomatrixes.

### EXPERIMENT

DNA constructs carrying the gene for beta-galactosidase were encapsulated during synthesis of nanomatrixes using the method of tube 3 of Experiment 15. Approximately 0.5 ml of the suspension of nanomatrixes were injected via the tail vein of mice. After 4 days, the mice were sacrificed and the blood cells were analyzed for expression of intracellular beta-galactosidase activity. Multiple cell types turned blue on exposure to X-gal chromogen, the colorless substrate, indicating the successful integration of the delivered gene to cells inside a live animal. Blood cells from control animals injected with either nanomatrixes without the beta-galactosidase gene or with unprotected naked beta-galactosidase DNA showed no expression of this enzyme.

Additional experiments were done by injection of 0.1 ml of the same suspension directly into the bone marrow of mice. Blood cells were harvested subsequently which again indicated transformation via only those nanomatrixes containing the beta-galactosidase gene construct.

The above experiments were repeated using beta-galactosidase gene nanomatrixes which had glycoprotein-120 attached to their surfaces. The results were consistent with an expected increased efficiency of transformation of blood cells.

### CONCLUSION

The DNA-containing nanomatrixes produced by the method of the present invention can perform successful transformation of cells in live animals.

### EXPERIMENT TWENTY

### TITLE Interaction of ribozymes delivered by nanomatrixes inside cells with ribozyme-substrate delivered also by nanomatrixes.

### EXPERIMENT

Ribozymes (RNA) were packaged during synthesis according to the method of Experiment 15 tube 2 and tube 3. RNA molecules which serve as substrate (with sequences similar to the target site of the HIV genome) were similarly entrapped. In vitro mixing of these two kinds of nanomatrixes resulted in no significant degradation of the substrate. 3T3 cells were cultured and exposed to nanomatrixes containing the ribozyme-substrate. Subsequently the cells were lysed in RNA-inhibiting media and the ribozyme-substrate were found not to be substantially degraded. However, when 3T3 cells which were exposed to ribozyme-substrate nanomatrixes were subsequently also exposed to ribozyme-nanomatrixes, ribozyme-substrate molecules were degraded. This shows that both RNA species were released intracellularly where they interact resulting in the degradation of the substrate. Ribozymes (RNA) or ribozyme-substrates added to cultures without nanomatrixes were degraded before they could enter the cells.

### CONCLUSION

The nanomatrixes produced by the method of the present invention can deliver ribozymes and ribozyme-substrate.

### EXPERIMENT TWENTY-ONE

### TITLE Protection of cells against infection of HIV by prior ingestion of nanomatrixes containing a ribozyme gene DNA construct.

### EXPERIMENT

Nanomatrixes containing two information molecules were constructed according to the conditions of tube 3 in Experiment 15: (a) DNA which carries the information of the ribozyme gene; and (b) DNA which carries the information of the beta-galactosidase. In addition, the reaction mixture also contained the glycoprotein 120 as a targeting molecule. Freshly isolated human CD₄ positive cells were activated and then incubated for at least 24 hours with these nanomatrixes, after which the beta-galactosidase substrate was added. Beta-galactosidase positive cells were sorted by a cell sorter. These cells were subsequently challenged by a HIV inoculum. HIV titers produced by infected cells were assayed on following days. The results were compared to CD₄ positive activated cells treated with control nanomatrixes with only the beta-galactosidase genes but no ribozyme gene. The result showed a protective effect from expression of the ribozyme gene delivered by the nanomatrixes against HIV infection. Addition of DNA alone without nanomatrixes resulted in no protective effect on cells.

### CONCLUSION

The nanomatrixes produced by the method of the present invention can deliver ribozyme gene DNA construct to human CD₄ positive cells, which produces a protection effect against infection of HIV.

### EXPERIMENT TWENTY-TWO

### TITLE Decrease viral load in cells previously infected with HIV by subsequent ingestion of nanomatrixes containing the ribozyme DNA construct.

### EXPERIMENT

Human macrophases previously infected with HIV were exposed to either (a) nanomatrixes containing both the DNA for the ribozyme and the beta galactosidase gene; or (b) control nanomatrixes with only the beta-galactosidase gene, both synthesized according to the conditions in Experiment 15 tube 3 in the presence of gp 120. Subsequent HIV titer analysis revealed results which were believed to be consistent with a much reduced titer of HIV only in cells treated with nanomatrixes containing the ribozyme gene. DNA added to cultures without nanomatrixes did not result in reduced HIV titer from infected cells.

### CONCLUSION

The nanomatrixes produced by the method of the present invention can deliver ribozyme gene DNA construct to human macrophases previously infected by HIV, which produces an effect to reduce HIV viral load.

### EXPERIMENT TWENTY-THREE

### TITLE Percentage of hemoglobin in nanomatrixes as compared to percentage of hemoglobin in solution before addition of alcohol.

### EXPERIMENT

Hemoglobin solutions (60 mg/ml) were mixed with human serum albumin (250 mg/ml) in various ratios as indicated below. Percent hemoglobin was calculated as milligram of hemoglobin divided by milligram of total protein (hemoglobin plus albumin), multiplied by 100.

| Tube | hemoglobin | albumin | normal saline | Calculated Percent hemoglobin |
|---|---|---|---|---|
| 1 | 0.050 ml | 0.450 ml | 0.500 ml | 2.6 |
| 2 | 0.075 ml | 0.425 ml | 0.500 ml | 4.1 |
| 3 | 0.100 ml | 0.400 ml | 0.500 ml | 5.7 |
| 4 | 0.125 ml | 0.375 ml | 0.500 ml | 7.4 |
| 5 | 0.150 ml | 0.350 ml | 0.500 ml | 9.3 |

Nanomatrixes were subsequently prepared as follows: Approximately 0.4 ml of ethanol was added to each of the five tubes to form nanomatrixes. After waiting 10 minutes, additional amounts of normal saline was added to make total volume to 4 ml per tube. Nanomatrixes were then removed from the suspensions by centrifugation The concentration of hemoglobin in the supernatant was measured by spectrophotometric methods at wavelength 416. Total protein concentration in the supernatant was measured by standard protein assays, read at wavelength 570.

| Tube | Percent of hemoglobin in supernatant |
|---|---|
| 1 | 1.63 |
| 2 | 3.05 |
| 3 | 3.93 |
| 4 | 5.67 |
| 5 | 8.28 |

This experiment also provided data on the yield of nanomatrixes, which is defined as: milligram of nanomatrixes formed divided by milligram of protein in the solution before addition of alcohol.

| Tube | Yield calculated | on the basis of |
|---|---|---|
| | Hemoglobin | Total protein |
| 1 | 0.758 | 0.775 |
| 2 | 0.779 | 0.758 |
| 3 | 0.792 | 0.752 |
| 4 | 0.655 | 0.757 |
| 5 | 0.638 | 0.753 |

### CONCLUSION

Since the percentage of hemoglobin left in the supernatant is similar to the percentage of hemoglobin in the solution before addition of alcohol (within experimental errors), the ratio of hemoglobin to albumin within the matrix of the nanomatrixes must also be similar to the hemoglobin to albumin ratio in the solution before addition of alcohol. Substantial mounts of both proteins are incorporated into the nanomatrixes. In other words, the albumin molecules are forming the interior of the nanomatrixes also, and not just coating the surface of spheres made up of a core of almost pure hemoglobin. Similarly, the data rejects a model of nanomatrixes which have hemoglobin molecules forming a coating layer around a core of almost pure albumin molecules.

### EXPERIMENT TWENTY-FOUR

### TITLE DNA molecules are protected by incorporation in the interior or by attachment on the surface of nanomatrixes from the degradation by endonucleases.

### EXPERIMENT

Nanomatrixes with DNA incorporated inside (DNA-in-spheres) or with DNA attached on the outside (DNA-out-spheres) were synthesized as follows:

### 1. DNA-in-spheres

Five microliters of hemoglobin solution (60 mg/ml) is mixed with 20 microliters of human serum albumin (250 mg/ml) and 20 microliters of DNA solution (containing 12.5 microgram of DNA). 18 microliters of ethanol was quickly added to result in 63 microliters of suspension containing nanomatrixes. Analysis of the DNA content in the supernatant and inside the nanomatrixes showed that 90% of all added DNA is associated with the nanomatrixes. The yield of nanomatrixes was approximately 20%. This means that at least 11.25 micrograms of DNA can be captured by 1.06 mg of nanomatrixes. The upper limits of DNA capable of being captured per nanomatrixes is not known yet due to the high cost of DNA and the low concentration of the DNA preparations available commercially. It is believed that one milligram of nanomatrixes can bind up to one milligram of DNA.

### 2. DNA-out-spheres

Five microliters of hemoglobin solution (60 mg/ml) is mixed with 20 microliter of human serum albumin (250 mg/ml). Ten microliters of ethanol was quickly added to result in 35 microliters of suspension containing nanomatrixes. 20 microliters of DNA solution (containing 12.5 microgram of DNA) was then added to the nanomatrix suspension. Analysis of the DNA content in the supernatant and DNA associated with the nanomatrixes showed that again 90% of all added DNA is associated with the nanomatrixes. The yield of nanomatrixes was also approximately 20%. This means that at least 11.25 micrograms of DNA can be captured by 1.06 mg of nanomatrixes. The upper limits of DNA per nanomatrixes obtained by this method has not been obtained yet due to the limited amount of DNA that is available and the low concentration of the DNA preparations available commercially. It is believed that one milligram of nanomatrixes can bind up to one milligram of DNA.

To show protection from the degradation by endocleases, aliquots of DNA in-spheres and DNA out-spheres (approximately 10 microliters each) were incubated with an endonuclease preparation for 30 minutes at 37 degrees centigrade. Subsequently, the suspensions were centrifuged to separate the supernatant from the nanomatrixes (which formed pellets in the centrifuge tubes). Migration patterns of the DNA bands (from DNA in the supernatant) in gel electrophoresis are consistent with DNA pieces degraded at the predicted endonuclease sites. The pellets were treated as follows: 50 microliters of EDTA (ethylenediaminetetraacetic acid) solution (50 millimolar) were layered onto the pellets carefully without disturbing the pellets. The presence of EDTA, a chelating agent, stops the activity of any endonucleases left over so that any DNA subsequently released from their association with the nanomatrixes will not by degraded after the nanomatrixes were solubilized. Five microliters of a weak alkaline solution (0.003 normal NaOH) were added to each of the DNA-in-sphere pellet and the DNA-out-sphere pellet. After incubation for 10 minutes at 37 degrees centigrade, five microliters of SDS (10 mg/ml) solution were added and the previous turbid suspensions became clear, indicating that the protein molecules constituting the nanomatrixes came apart again and the nanomatrixes were completely solubilized. Migration patterns of the released DNA (from both DNA-in-spheres and DNA-out-spheres) in gel electrophoresis were identical to control naked DNA molecules which were not degraded.

### CONCLUSION

Whether DNA is hidden in the interior of the nanomatrixes or attached on the surface of the nanomatrixes, they became protected from enzymatic degradation. Large amounts of DNA can be protected by these methods. The mechanisms of protection is not known. It is possible that DNA vulnerable sites were covered by the protein molecules so that the endonucleases cannot reach them, or the stereohinderance presented by the DNA's association with the nanomatrixes prevents the proper orientation of the endonucleases or the DNA molecules. Alternatively, co-factors needed by the degradative process may not be available on the surface or interior of nanomatrixes for the enzymatic action to proceed. Nanomatrixes can be solubilized easily by agents not known to break covalent bonds. DNA incorporated in the interior or attached on the surface of the nanomatrixes were not damaged in any way and could be released again in intact form from these reversible nanomatrixes.

The experiments of the present invention have discovered the following important facts:
a. Pure hemoglobin (Hb) nanomatrixes could be synthesized in a normal saline buffer but they are unstable and tend to aggregate together. Presence of human serum albumin (HSA) of approximately 4% (weight of HSA per total weight of protein) will provide stability to the primarily Hb nanomatrixes (about 96% of total protein mass). A higher HSA to hemoglobin ratio also resulted to form progressively smaller nanomatrixes.
b. Pure Hb nanomatrixes could be also synthesized in water but they are unstable and tend to aggregate together. Presence of at least one of the stabilizer such as sodium lauryl sulphate (STS) or Gelatin will provide stability to the primarily Hb nanomatrixes.
c. A higher gelatin to hemoglobin ratio resulted in smaller nanomatrixes. No significant difference in size was observed by using a higher HSA to hemoglobin ratio on nanomatrixes synthesized in butanol, but differences in size are observed by using a higher HSA to hemoglobin ratio on nanomatrixes synthesized in propanol.
d. Higher concentrations of hemoglobin solution resulted in larger nanomatrixes, and at least 4.76 percent by weight of gelatin is needed for preventing aggregates occurring in synthesis of Hb nanomatrixes.
e. The absence of a stabilizer such as hemoglobin from HSA solution resulted nanomatrixes that can easily be reversed by dilution of reagents. However, inclusion of as little as 1.48% of hemoglobin will greatly increase the stability of the HSA nanomatrixes.
f. Substantially high concentration of surfactant resulted in larger HSA nanomatrix size, excessive surfactant even resulted in aggregates. When a critical amount of neutral organic solvent was exceeded by a small amount, the result was aggregated spheres instead of monodispersed nanomatrixes. The highest final concentration of non-dialyzed HSA (before addition of neutral organic solvent and in the absence of Hb) that can produce monodispersed nanomatrixes in the absence of Hb is 148 mg/ml. With Dialyzed HSA the final concentration that can be used to produce monodispersed nanomatrixes in the absence of Hb may be higher. Also methanol tends to produce smaller spheres than ethanol. It may be possible that a low STS concentration such as 1.4 mg/ml in conjunction with methanol would produce useful nanomatrixes with final concentrations of dialyzed HSA even higher than 148 mg/ml. Different neutral organic solvents have different effects on the sizes of Hb and HSA nanomatrixes.
g. Hb and HSA nanomatrixes encapsulating drugs are stable. Also the biological agents pre-bonded to protein molecules can be incorporated into Hb nanomatrixes.
h. Biologically active molecules entrapped by Hb and HSA nanomatrixes can maintain their biological activities.
i. Substantially higher final concentrations of non-dialyzed HSA can be used (e.g. 200mg/ml.) before addition of neutral organic solvent in the present of Hb (e.g. 12 mg/ml) to produce monodispersed nanomatrixes.

## Claims

1. A method of producing monodispersed nanomatrixes for biologically active molecules to bind to or be trapped therein, useful for carrying medication for in vivo administration, which nanomatrixes are stable in the synthetic media but are readily reversible to soluble protein molecules after in vivo administration, the method comprising:
a. dissolving first protein molecules in a buffer of suitable osmolarity between approximately 1 to 970±100 milliosmos, where the first protein molecules are globin protein molecules;
b. adding second protein molecules into said buffer containing said first protein molecules, such that the weight ratio of said first protein molecules and the second protein molecules is approximately 96:4, where the second protein molecules are different from said first protein molecules and selected from the group consisting of albumin protein and denatured hypoallergic protein; and
c. adding a solution containing an organic solvent into said buffer containing said first and second protein molecules, such that the organic solvent is approximately 0.2±0.1 to 2.0±0.1 volume by one volume of said buffer containing said first and second protein molecules, where said organic solvent is an alcohol selected from the group consisting of methanol, ethanol, propanol and butanol;
d. whereby mixing said buffer containing said first and second protein molecules and said solution containing said organic solvent results in a turbid suspension containing monodispersed stable nanomatrixes.

2. A method of producing stable nanomatrixes as defined in Claim 1 wherein said globin protein molecules are hemoglobin molecules.

3. A method of producing nanomatrixes as defined in Claim 1 wherein said second protein molecules are gelatin molecules.

4. A method of producing stable nanomatrixes as defined in Claim 1 further comprising adding biologically active or interactive molecules into said buffer containing said first and second protein molecules before or after adding said solution containing said organic solvent, such that the weight ratio of said first protein molecules to said second protein molecules to the biologically active or interactive molecules is approximately 96:4:25.

5. A method of producing nanomatrixes as defined in Claim 1 wherein said first or said second protein molecules comprise protein molecules covalently bonded with biologically active or interactive molecules respectively, so that said buffer containing said first and second protein molecules also contains the biologically active or interactive molecules, such that the weight ratio of said first protein molecules to said second protein molecules to the biologically active or interactive molecules is approximately 96:4:25.

6. A method of producing nanomatrixes as defined in Claim 1 further comprising finally separating said nanomatrixes from said buffer by a method or a combination of methods selected from the group consisting of dialysis, centrifugation, ultrafiltration, electrodialysis, absorption to solid surface and chromatography.

7. A composition of monodispersed nanomatrixes useful for carrying medication for in vivo administration, where the nanomatrixes are stable in the media in which said nanomatrixes were synthesized but are readily reversible to soluble protein molecules after in vivo administration, the device having a composition of,
a. a first protein ingredient being a globin protein;
b. a second protein ingredient which is different from said first protein ingredient, where the second protein ingredient is albumin protein or denatured hypoallergic protein; and
c. the weight ratio of said first protein ingredient and said second protein ingredient being approximately 96:4.

8. A composition of monodispersed nanomatrixes as defined in Claim 7 further comprising,
a. at least one biologically interactive ingredient; and
b. the weight ratio of the at least one biologically interactive ingredient to the sum of said protein ingredients is not more than approximately 25:100.

9. A composition of monodispersed nanomatrixes as defined in claims 7 or 8 wherein said second protein ingredient is gelatin protein.

10. A composition as claimed in any of claims 7 to 9 for use in therapy, said composition further comprising a biologically interactive ingredient.

## Patentansprüche

1. Verfahren zur Herstellung monodisperser Nanomatrizen, damit sich biologisch wirksame Moleküle daran binden oder darin eingeschlossen werden, wobei sich die Nanomatrizen zum Transport von Medikamenten zur Verabreichung in vivo eignen, und wobei die Nanomatrizen in den synthetischen Trägern stabil sind, nach der Verabreichung in vivo aber leicht in lösliche Proteinmoleküle umgewandelt werden können, wobei das Verfahren die folgenden Schritte umfaßt:
a. Lösen erster Proteinmoleküle in einem Puffer mit einer geeigneten Osmolarität von ungefähr 1 bis 970±100 mOsmol, wobei die ersten Proteinmoleküle Globinproteinmoleküle sind;
b. Zugabe zweiter Proteinmoleküle zu dem die ersten Proteinmoleküle enthaltenden Puffer, so daß das Gewichtsverhältnis der ersten Proteinmoleküle zu den zweiten Proteinmolekülen ungefähr 96:4 beträgt, wobei die zweiten Proteinmoleküle von den ersten Proteinmolekülen verschieden sind und ausgewählt sind aus der Gruppe umfassend Albuminprotein und denaturiertes hypoallergenes Protein; und
c. Zugabe einer Lösung, die ein organisches Lösungsmittel enthält, zu dem Puffer die ersten und zweiten Proteinmoleküle enthaltenden Puffer, so daß das organische Lösungsmittel ungefähr 0,2±0,1 bis 2,0±0,1 Volumen pro einem Volumen des die ersten und zweiten Proteinmoleküle enthaltenden Puffers darstellt, wobei das organische Lösungsmittel ein Alkohol ist, der ausgewählt ist aus der Gruppe umfassend Methanol, Ethanol, Propanol und Butanol;
d. so daß man durch Mischen des die ersten und zweiten Proteinmoleküle enthaltenden Puffers mit der das organische Lösungsmittel enthaltenden Lösung eine trübe Suspension erhält, die monodisperse stabile Nanomatrizen enthält.

2. Verfahren zur Herstellung stabiler Nanomatrizen nach Anspruch 1, bei dem die Globinproteinmoleküle Hämoglobinmoleküle sind.

3. Verfahren zur Herstellung von Nanomatrizen nach Anspruch 1, bei dem die zweiten Proteinmoleküle Gelatinemoleküle sind.

4. Verfahren zur Herstellung stabiler Nanomatrizen nach Anspruch 1, des weiteren umfassend die Zugabe biologisch wirksamer bzw. interaktiver Moleküle zu dem die ersten und zweiten Proteinmoleküle enthaltenden Puffer, bevor oder nachdem die das organische Lösungsmittel enthaltende Lösung zugegeben wird, so daß das Gewichtsverhältnis der ersten Proteinmoleküle zu den zweiten Proteinmolekülen zu den biologisch wirksamen bzw. interaktiven Molekülen ungefähr 96:4:25 beträgt.

5. Verfahren zur Herstellung von Nanomatrizen nach Anspruch 1, bei dem die ersten oder zweiten Proteinmoleküle Proteinmoleküle umfassen, die kovalent an biologisch wirksame bzw. interaktive Moleküle gebunden sind, so daß der die ersten und zweiten Proteinmoleküle enthaltende Puffer auch die biologisch wirksamen bzw. interaktiven Moleküle enthält, so daß das Gewichtsverhältnis der ersten Proteinmoleküle zu den zweiten Proteinmolekülen zu den biologisch wirksamen bzw. interaktiven Molekülen ungefähr 96:4:25 beträgt.

6. Verfahren zur Herstellung von Nanomatrizen nach Anspruch 1, bei dem des weiteren die Nanomatrizen schließlich von dem Puffer getrennt werden durch ein Verfahren oder eine Kombination von Verfahren, die ausgewählt sind aus der Gruppe umfassend Dialyse, Zentrifugieren, Ultrafiltration, Elektrodialyse, Absorption auf feste Oberfläche, und Chromatographie.

7. Zusammensetzung aus monodispersen Nanomatrizen zum Transport von Medikamenten für die Verabreichung in vivo, wobei die Nanomatrizen in Trägern stabil sind, in denen die Nanomatrizen synthetisiert wurden, nach der Verabreichung in vivo aber leicht in lösliche Proteinmoleküle umgewandelt werden können, wobei die Vorrichtung die folgende Zusammensetzung aufweist:
a. einen ersten Proteinbestandteil, bei dem es sich um ein Globinprotein handelt;
b. einen zweiten Proteinbestandteil, der von dem ersten Proteinbestandteil verschieden ist, wobei es sich bei dem zweiten Proteinbestandteil um Albuminprotein oder denaturiertes hypoallergenes Protein handelt; und
c. wobei das Gewichtsverhältnis des ersten Proteinbestandteils zu dem zweiten Proteinbestandteil ungefähr 96:4 beträgt.

8. Zusammensetzung aus monodispersen Nanomatrizen nach Anspruch 7, des weiteren umfassend:
a. mindestens einen biologisch interaktiven Bestandteil; und
b. das Gewichtsverhältnis des mindestens einen biologisch interaktiven Bestandteils zu der Summe der Proteinbestandteile beträgt nicht mehr als ungefähr 25:100.

9. Zusammensetzung aus monodispersen Nanomatrizen nach Anspruch 7 oder 8, bei der der zweite Proteinbestandteil Gelatineprotein ist.

10. Zusammensetzung nach einem der Ansprüche 7 bis 9 für Therapiezwecke, wobei die Zusammensetzung des weiteren einen biologisch interaktiven Bestandteil umfaßt.

## Revendications

1. Procédé de production de nanomatrices monodispersées liant ou piégeant dans leur masse des molécules biologiquement actives, lesquelles nanomatrices sont stables dans un milieu synthétique mais sont facilement réversibles en molécules protéiniques solubles après administration in vivo, ledit procédé comprenant les étapes consistant à :
a. dissoudre des premières molécules protéiniques dans un tampon ayant une osmoralité appropriée approximativement comprise entre 1 à 970±100 milliosmos, où les premières molécules protéiniques sont des molécules protéiniques de globine ;
b. ajouter des secondes molécules protéiniques dans ledit tampon contenant lesdites premières molécules protéiniques, de telle façon que le rapport pondéral desdites premières molécules protéiniques aux secondes molécules protéiniques soit approximativement de 96/4, où les secondes molécules protéiniques sont différentes desdites premières molécules protéiniques et sont choisies parmi l'ensemble constitué par la protéine d'albumine et la protéine dénaturée hypoallergénique ; et
c. ajouter une solution contenant un solvant organique dans ledit tampon contenant les premières et secondes molécules protéiniques, de telle façon que le solvant organique représente approximativement 0,2±0,1 à 2,0±0,1 volume pour 1 volume dudit tampon contenant les premières et secondes molécules protéiniques, où ledit solvant organique est un alcool choisi parmi l'ensemble constitué par les méthanol, éthanol, propanol et butanol ;
d. le mélange dudit tampon contenant lesdites premières et secondes molécules protéiniques et de ladite solution contenant ledit solvant organique fournissant une suspension trouble contenant des nanomatrices stables monodispersées.

2. Procédé de production de nanomatrices stables suivant la revendication 1, dans lequel lesdites molécules protéiniques de globine sont des molécules d'hémoglobine.

3. Procédé de production de nanomatrices stables suivant la revendication 1, dans lequel lesdites secondes molécules protéiniques sont des molécules de gélatine.

4. Procédé de production de nanomatrices stables suivant la revendication 1, comprenant en outre l'ajout de molécules biologiquement actives ou interactives dans ledit tampon contenant lesdits premières et secondes molécules protéiniques avant ou après l'ajout de ladite solution contenant ledit solvant organique, de telle façon que le rapport pondéral dites premières molécules protéiniques/dites secondes molécules protéiniques/molécules biologiquement actives ou interactives soit approximativement de 96/4/25.

5. Procédé de production de nanomatrices stables suivant la revendication 1, dans lequel lesdites premières ou dites secondes molécules protéiniques comprennent des molécules protéiniques liées par covalence avec des molécules biologiquement actives ou interactives respectivement, de sorte que ledit tampon comprenant lesdites premières et secondes molécules protéiniques contient aussi les molécules biologiquement actives ou interactives, de telle façon que le rapport pondéral dites premières molécules protéiniques/dites secondes molécules protéiniques/molécules biologiquement actives ou interactives soit approximativement de 96/4/25.

6. Procédé de production de nanomatrices stables suivant la revendication 1, comprenant en outre la séparation finale desdites nanomatrices dudit tampon par une méthode ou une combinaison de méthodes choisies parmi l'ensemble constitué par les dialyse, centrifugation, ultrafiltration, électrodialyse, absorption sur une surface solide et chromatographie.

7. Composition de nanomatrices monodispersées utiles pour véhiculer une médication pour administration *in vivo* dans laquelle les nanomatrices sont stables dans le milieu dans lequel lesdites nanomatrices ont été synthétisées mais sont facilement réversibles en molécules protéiniques solubles après administration *in vivo*, ladite composition comprenant dans sa formulation
a. un premier ingrédient protéinique qui est une protéine de globine ;
b. un second ingrédient protéinique qui est différent du premier ingrédient protéinique, le second ingrédient protéinique étant une protéine d'albumine ou une protéine dénaturée hypoallergénique ; et
c. un rapport pondéral desdits premier et second ingrédients protéiniques qui est approximativement de 96/4.

8. Composition de nanomatrices monodispersées suivant la revendication 7, comprenant en outre,
a. au moins un ingrédient biologiquement interactif ; et
b. le rapport pondéral dudit ingrédient biologiquement interactif à la somme totale desdits ingrédients protéiniques n'étant pas approximativement supérieur à 25/100.

9. Composition de nanomatrices monodispersées suivant la revendication 7 ou 8, dans laquelle le second ingrédient protéinique est la protéine de gélatine.

10. Composition suivant l'une quelconque des revendications 7 à 9, pour utilisation en thérapeutique, ladite composition comprenant en outre un ingrédient biologiquement interactif.
